# EUROPEAN PATENT APPLICATION

(11) **EP 3 498 711 A1**
(43) Date of publication of application: **19.06.2019**
(21) Application number: 17839452.4
(22) Date of filing: 08.08.2017
(51) Int. Cl.: C07D 487/08, A61K 31/439, A61P 13/10, A61P 25/04, A61P 25/16, A61P 25/22, A61P 25/24, A61P 27/06, C07D 491/18

(54) **MORPHINAN DERIVATIVE**

(30) Priority: 09.08.2016 JP 2016156049
(71) Applicant: Nippon Chemiphar Co., Ltd., Tokyo 101-0032 (JP)
(72) Inventor: NAGASE, Hiroshi, Tsukuba-shi Ibaraki 305-8577 (JP); FUJII, Hideaki, Tokyo 108-8641 (JP); SAITOH, Akiyoshi, Kodaira-shi Tokyo 187-8551 (JP); NAKATA, Eriko, Misato-shi Saitama 341-0005 (JP); HIROSE, Masaaki, Misato-shi Saitama 341-0005 (JP); OOI, Isao, Misato-shi Saitama 341-0005 (JP); HAYASHIDA, Kohei, Misato-shi Saitama 341-0005 (JP)
(74) Representative: Elkington and Fife LLP
(86) International application number: PCT/JP2017/028709
(87) International publication number: WO 2018/030382

(57) **Abstract**

A compound represented by the following general formula (I), 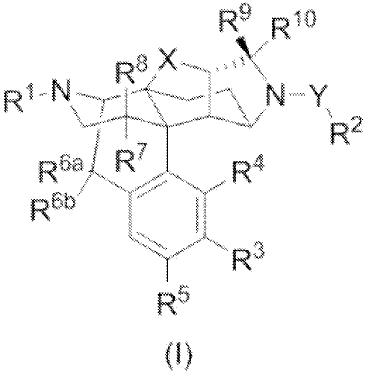 wherein R¹ represents hydrogen, C₁₋₁₀ alkyl, cycloalkylalkyl where the cycloalkyl moiety has 3 to 6 carbon atoms and the alkylene moiety has 1 to 5 carbon atoms, or the like,
R² represents a 4- to 7-membered saturated heterocycle containing one or two heteroatoms which may be the same or different and are selected from N, O, and S, and two or more carbon atoms as ring-constituting atoms,
the heterocycle may be substituted with a substituent such as an oxo group,
R² binds to Y via a carbon atom as a ring-constituting atom of R²,
R³, R⁴, and R⁵, which are the same or different, represent hydrogen; hydroxy; or the like,
R^{6a} and R^{6b}, which are the same or different, represent hydrogen or the like,
R⁷ and R⁸, which are the same or different, represent hydrogen or the like,
R⁹ and R¹⁰, which are the same or different, represent hydrogen or the like,
X represents O or CH₂, and
Y represents C(= O) or the like),
a tautomer of the compound, a stereoisomer of the compound, a pharmaceutically acceptable salt thereof, or a solvate thereof is used as an anxiolytic, an antidepressant, or the like.

## Description

### Technical Field

The present invention relates to a morphinan derivative having an opioid δ receptor agonistic effect.

### Background Art

Opioids bind to opioid receptors to exhibit the effect thereof, and there are three kinds of subtypes of the opioid receptors, i.e., µ, δ, and κ receptors. It is known that agonists of each of the three subtypes, i.e., µ, δ, and κ, have analgesic effects. For example, it is known that enkephalin which is an endogenous ligand of the opioid δ receptor has an analgesic effect.

However, although morphine, an agonist of the opioid µ receptor showing a high affinity to the receptor, has a potent analgesic effect, it also shows adverse effects such as dependence, drug abuse, tolerance, respiratory depression, constipation caused by suppression of gastrointestinal motility, nausea and vomiting, blood pressure reductions, bradycardia, cough reflex inhibition, and sleepiness.

Although eptazocine, a selective agonist of the opioid κ receptor, has a potent analgesic effect, and shows mild dependence, tolerance, sleepiness, constipation, and respiratory depression, it causes sweating, nausea and vomiting, and thirst.

It is also known that activation of the opioid δ receptor provides analgesic, antidepressive, and anxiolytic effects. For example, it is known that anxiety-like and depressive-like behaviors increase in receptor-deficient mice (Non Patent Literature 1) and that enhancement of an enkephalin-δ receptor system is involved in emotional regulation (Non Patent Literature 2). Further, since the antidepressive and anxiolytic-like-effects of various δ receptor agonists are antagonized by a δ receptor antagonist in various rat and mouse anxiety and depression models, usefulness of δ receptor-selective agonists as antidepressive and anxiolytic drugs have been demonstrated (Non-patent documents 3 to 7, Patent documents 1 and 2). It is expected that an agonist that selectively activates the opioid δ receptor does not show or scarcely shows adverse effects that are induced through activation of the opioid µ receptor or opioid κ receptor.

In addition, it is suggested that activation of the δ receptor shows effects for improving neurodegenerative diseases such as Parkinson's disease and Alzheimer's disease, ischaemia or cerebral stroke, urinary dysfunction, HIV infection, alcohol dependence, diabetes, and the like (Non-patent document 8). Various compounds have so far been reported as opioid δ agonists, and analgesic effects, antidepressive effects, and anxiolytic effects thereof have been verified (Patent documents 1 to 6, Non-patent document 9). It has also been reported that some opioid δ agonists such as SNC80 and BW373U86 induce convulsion (Non-patent documents 5, 6, and 10) .

As antidepressants, tetracyclic antidepressants and triazolopyridine type antidepressants have been developed in addition to the classic tricyclic antidepressants and monoamine oxidase inhibitors, and in recent years, selective serotonin reuptake inhibitors (SSRI), serotonin-noradrenalin reuptake inhibitors (SNRI), and noradrenergic and specific serotonergic antidepressants (NaSSA) are frequently used. However, effectiveness of all these antidepressants is not so high as evaluated in terms of remission rate. Usefulness thereof is also limitative, because of early development of increased aggression after start of administration, risk of suicidal ideation and suicide attempt of youth age patients, and the like.

As anxiolytic drugs, although benzodiazepine type drugs are widely used, this type of drugs have outstanding problems, for example, difficulty in use for elderly people and patients showing a bad general state, because of adverse effects of them such as dependence, hypnotic action, muscle relaxation, sedation, and cognitive function decline at regular dose. Although indications of SSRI and SNRI developed as antidepressants are recently expanded to various anxiety disorders, they do not show immediate effects, and also show adverse effects. Although anesthetic drugs such as barbiturate also show anxiolytic effects, effective dose and fatal dose thereof are close to each other, and therefore they are drugs having risks.

Therefore, it is desired to develop an anxiolytic and an antidepressant that show effects thereof through a mechanism different from those of the presently used drugs, and show improved adverse effects and safety.

### Citation List

### Patent Literature

Patent Literature 1: JP 2006-522775 A
Patent Literature 2: WO 2001/046192
Patent Literature 3: WO 2008/001859
Patent Literature 4: WO 2013/035833
Patent Literature 5: WO 2014/021273
Patent Literature 6: WO 2014/136305
Patent Literature 7: WO 2016/148232

### Non Patent Literature

Non Patent Literature 1: Nature Genetics 2000, 25, 195
Non Patent Literature 2: Neuroscience 2005, 135, 305
Non Patent Literature 3: J. Pharmacol. Exp. Ther. 2011, 338, 195
Non Patent Literature 4: Trends in Neurosciences 2013, 36, 195
Non Patent Literature 5: Behavioral Brain Research 2011, 223, 271
Non Patent Literature 6: Neuropharmacology, 2013, 67, 485
Non Patent Literature 7: Current Neuropharmacology, 2012, 10, 231
Non Patent Literature 8: Psychopharmacology (Berl) 2013, 228, 1
Non Patent Literature 9: Tetrahedron, 2011, 67, 6682
Non Patent Literature 10: The International Narcotics Research Conference 2014, July 13, 2014

### Summary of the Invention

### Problem to be Solved by the Invention

An object of the present invention is to provide an anxiolytic, an antidepressant, an analgesic drug, a therapeutic agent for Parkinson's disease, and a therapeutic agent for pollakiuria and urinary incontinence that are highly effective, show less adverse effects such as dependence, tolerance, respiratory depression, constipation, nausea and vomiting, blood pressure reductions, bradycardia, cough reflex inhibition, hypnotic effects, muscle relaxation, sedation, cognitive function decline, sweating, and thirst, and are safe. Another object of the present invention is to provide a safe medicament that can even simultaneously exhibit antidepressive, anxiolytic, and analgesic effects, and thereby provide good news to patients suffered from depression, anxiety, and pain. A further object of the present invention is to provide a medicament that can be used for simultaneously treating depression, anxiety, and pain as single medicament, and that can be safe and administered orally or by injection (for example, subcutaneous injection).

### Means for Solving the Problem

The present invention relates to a compound represented by the following general formula (I), 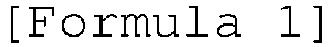 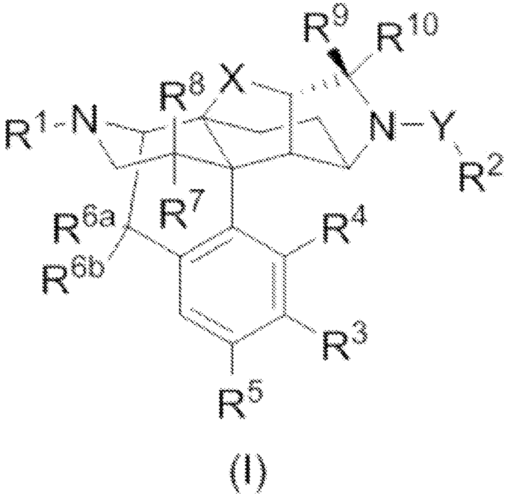
(wherein R¹ represents hydrogen; C1-10 alkyl; C₆₋₁₀ aryl; C₂₋₆ alkenyl; cycloalkylalkyl where the cycloalkyl moiety has 3 to 6 carbon atoms, and the alkylene moiety has 1 to 5 carbon atoms; aralkyl where the aryl moiety has 6 to 10 carbon atoms, and the alkylene moiety has 1 to 5 carbon atoms; C₃₋₆ cycloalkyl; or heteroarylalkyl where the heteroaryl moiety contains 1 to 4 heteroatoms selected from N, O and S as ring-constituting atoms, and the alkylene moiety has 1 to 5 carbon atomsring-constituting atom,
R² represents 4- to 7-membered saturated heterocycle ring containing one or two heteroatoms which may be the same or different and are selected from N, O, and S, and two or more carbon atoms as ring-constituting atomsring,
R² binds to Y via a carbon atom as a ring-constituting atom of R²,
R³, R⁴, and R⁵, which are the same or different, represent hydrogen; hydroxy; halogen; cyano; carbamoyl; C₁₋₆ alkoxy; C₆₋₁₀ aryloxy; C₁₋₆ alkanoyloxy; nitro; amino; C₁₋₈ alkylamino; C₆₋₁₀ arylamino; or acylamino where the acyl moiety has 2 to 6 carbon atoms,
R^{6a} and R^{6b}, which are the same or different, represent hydrogen; fluorine, or hydroxy, or R^{6a} and R^{6b} combine together to represent = O,
R⁷ and R⁸, which are the same or different, represent hydrogen; fluorine, or hydroxy,
R⁹ and R¹⁰, which are the same or different, represent hydrogen; C₁₋₆ alkyl; C₆₋₁₀ aryl; heteroaryl containing 1 to 4 heteroatoms selected from N, O, and S as ring-constituting atoms; aralkyl where the aryl moiety has 6 to 10 carbon atoms, and the alkylene moiety has 1 to 5 carbon atoms; heteroarylalkyl where the heteroaryl moiety contains 1 to 4 heteroatoms selected from N, O and S as ring-constituting atoms, and the alkylene moiety has 1 to 5 carbon atoms; cycloalkylalkyl where the cycloalkyl moiety has 3 to 6 carbon atoms, and the alkylene moiety has 1 to 5 carbon atoms; or C₂₋₆ alkenyl,
X represents O or CH₂,
Y represents C(= O); SO₂; C(= O)O; C(= O)NR¹¹; or an atomic bond, where R¹¹ represents hydrogen; C₁₋₁₀ alkyl; or aralkyl where the aryl moiety has 6 to 10 carbon atoms, and the alkylene moiety has 1 to 5 carbon atoms,
provided that the C₁₋₁₀ alkyl as R1; the alkylene moiety and cycloalkyl moiety of the cycloalkylalkyl where the cycloalkyl moiety has 3 to 6 carbon atoms, and the alkylene moiety has 1 to 5 carbon atoms as R¹; the alkylene moiety of the aralkyl where the aryl moiety has 6 to 10 carbon atoms, and the alkylene moiety has 1 to 5 carbon atoms as R¹; and the alkylene moiety of the heteroarylalkyl where the heteroaryl moiety contains 1 to 4 heteroatoms selected from N, O and S as ring-constituting atoms, and the alkylene moiety has 1 to 5 carbon atoms as R¹ may be substituted with at least one substituent selected from
1 to 6 halogens; hydroxy; C₁₋₆ alkoxy; C₆₋₁₀ aryloxy; C₁₋₆ alkanoyl; C₁₋₆ alkanoyloxy; carboxyl; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; alkylsulfonyl where the alkyl moiety has 1 to 6 carbon atoms; aminosulfonyl; alkylsulfinyl where the alkyl moiety has 1 to 6 carbon atoms; alkylthio where the alkyl moiety has 1 to 6 carbon atoms; C₁₋₆ alkoxy substituted with 1 to 6 halogens; and arylcarbonyl where the aryl moiety has 6 to 10 carbon atoms,
the C₆₋₁₀ aryl as R¹; the aryl moiety of the aralkyl where the aryl moiety has 6 to 10 carbon atoms, and the alkylene moiety has 1 to 5 carbon atoms as R¹; the aryl moiety of the C₆₋₁₀ aryloxy as R³, R⁴, or R⁵; the aryl moiety of the C₆₋₁₀ arylamino as R³, R⁴, or R⁵; the C₆₋₁₀ aryl as R⁹ or R¹⁰; the heteroaryl containing 1 to 4 heteroatoms selected from N, O and S as ring-constituting atoms as R9 or R10; the aryl moiety of the aralkyl where the aryl moiety has 6 to 10 carbon atoms, and the alkylene moiety has 1 to 5 carbon atoms as R9 or R10; and the heteroaryl moiety of the heteroarylalkyl where the heteroaryl moiety contains 1 to 4 heteroatoms selected from N, O and S as ring-constituting atoms, and the alkylene moiety has 1 to 5 carbon atoms as R⁹ or R¹⁰ may be substituted with at least one substituent selected from
C₁₋₆ alkyl; C₁₋₆ alkoxy; C₁₋₆ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; C₁₋₆ alkyl substituted with 1 to 3 halogens; C₁₋₆ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy,
the heterocyclic ring as R² may have, besides the oxo group, the substituents that the C₆₋₁₀ aryl as R¹ mentioned above may have,
when R¹ is C₁₋₁₀ alkyl, it may be substituted with NR¹¹R¹², where R¹¹ and R¹², which are the same or different, represent hydrogen; C₁₋₁₀ alkyl; or aralkyl where the aryl moiety has 6 to 10 carbon atoms, and the alkylene moiety has 1 to 5 carbon atoms; or R¹¹, R¹², the nitrogen atom to which R¹¹ and R¹² bind, and optionally, 1 or 2 heteroatoms may combine together to form a 5- to 7-membered ring, and
the alkylene moiety of the aralkyl where the aryl moiety has 6 to 10 carbon atoms, and the alkylene moiety has 1 to 5 carbon atoms as R¹ may be substituted with at least one substituent selected from phenyl, and C1-6 alkyl substituted with 1 to 3 halogens),
a tautomer of the compound, a stereoisomer of the compound, a pharmaceutically acceptable salt thereof, or a solvate thereof.

The present invention also relates to a medicament comprising a compound represented by the general formula (I), a tautomer of the compound, a stereoisomer of the compound, a pharmaceutically acceptable salt thereof, or a solvate thereof.

The present invention also relates to a pharmaceutical composition comprising a compound represented by the general formula (I), a tautomer of the compound, a stereoisomer of the compound, a pharmaceutically acceptable salt thereof, or a solvate thereof as an active ingredient.

The present invention also relates to an analgesic comprising a compound represented by the general formula (I), a tautomer of the compound, a stereoisomer of the compound, a pharmaceutically acceptable salt thereof, or a solvate thereof as an active ingredient.

The present invention also relates to an antidepressant comprising a compound represented by the general formula (I), a tautomer of the compound, a stereoisomer of the compound, a pharmaceutically acceptable salt thereof, or a solvate thereof as an active ingredient.

The present invention also relates to an anxiolytic comprising a compound represented by the general formula (I), a tautomer of the compound, a stereoisomer of the compound, a pharmaceutically acceptable salt thereof, or a solvate thereof as an active ingredient.

The present invention also relates to a method for ameliorating, preventing, or treating depression, the method comprising administering an effective amount of a compound represented by the general formula (I), a tautomer of the compound, a stereoisomer of the compound, a pharmaceutically acceptable salt thereof, or a solvate thereof.

The present invention also relates to a method for ameliorating, preventing, or treating anxiety, the method comprising administering an effective amount of a compound represented by the general formula (I), a tautomer of the compound, a stereoisomer of the compound, a pharmaceutically acceptable salt thereof, or a solvate thereof.

The present invention also relates to a method for ameliorating, preventing, or treating pain, the method comprising administering an effective amount of a compound represented by the general formula (I), a tautomer of the compound, a stereoisomer of the compound, a pharmaceutically acceptable salt thereof, or a solvate thereof.

The present invention also relates to use of a compound represented by the general formula (I), a tautomer of the compound, a stereoisomer of the compound, a pharmaceutically acceptable salt thereof, or a solvate thereof for ameliorating, preventing, or treating pain, depression, or anxiety.

The present invention also relates to a method for ameliorating, preventing, or treating pain, depression, or anxiety, the method comprising administering an effective amount of a compound represented by the general formula (I), a tautomer of the compound, a stereoisomer of the compound, a pharmaceutically acceptable salt thereof, or a solvate thereof to a human.

The present invention also relates to an agent for treating Parkinson's disease, the agent comprising a compound represented by the general formula (I), a tautomer of the compound, a stereoisomer of the compound, a pharmaceutically acceptable salt thereof, or a solvate thereof as an active ingredient.

The present invention also relates to a method for ameliorating, preventing, or treating Parkinson's disease, the method comprising administering an effective amount of a compound represented by the general formula (I), a tautomer of the compound, a stereoisomer of the compound, a pharmaceutically acceptable salt thereof, or a solvate thereof.

The present invention also relates to use of a compound represented by the general formula (I), a tautomer of the compound, a stereoisomer of the compound, a pharmaceutically acceptable salt thereof, or a solvate thereof for ameliorating, preventing, or treating Parkinson's disease.

The present invention also relates to a method for ameliorating, preventing, or treating Parkinson's disease in a human, the method comprising administering an effective amount of a compound represented by the general formula (I), a tautomer of the compound, a stereoisomer of the compound, a pharmaceutically acceptable salt thereof, or a solvate thereof to a human.

The present invention also relates to an agent for treating pollakiuria or urinary incontinence, the agent comprising a compound represented by the general formula (I), a tautomer of the compound, a stereoisomer of the compound, a pharmaceutically acceptable salt thereof, or a solvate thereof as an active ingredient.

The present invention also relates to a method for ameliorating, preventing, or treating pollakiuria or urinary incontinence, the method comprising administering an effective amount of a compound represented by the general formula (I), a tautomer of the compound, a stereoisomer of the compound, a pharmaceutically acceptable salt thereof, or a solvate thereof.

The present invention also relates to use of a compound represented by the general formula (I), a tautomer of the compound, a stereoisomer of the compound, a pharmaceutically acceptable salt thereof, or a solvate thereof for ameliorating, preventing, or treating pollakiuria or urinary incontinence.

The present invention also relates to a method for ameliorating, preventing, or treating pollakiuria or urinary incontinence in a human, the method comprising administering an effective amount of a compound represented by the general formula (I), a tautomer of the compound, a stereoisomer of the compound, a pharmaceutically acceptable salt thereof, or a solvate thereof to a human.

### Effects of the Invention

The compounds represented by the general formula (I), tautomers of the compounds, stereoisomers of the compounds, pharmaceutically acceptable salts thereof, and solvates thereof, which are the compounds provided by the present invention, exhibit potent agonistic activity for the opioid δ receptor, but do not activate or only extremely weakly activate the µ and κ receptors, and therefore they have superior antidepressive effects, anxiolytic effects and analgesic effects based on activation of the opioid δ receptor. Since the compounds of the present invention do not activate or only extremely weakly activate the µ and κ receptors, they do not provide or extremely weakly provide adverse effects such as dependence, drug abuse, tolerance, respiratory depression, constipation caused by suppression of gastrointestinal motility, nausea and vomiting, blood pressure reductions, bradycardia, cough reflex inhibition, sleepiness, sweating, and thirst. As far as the inventors of the present invention examined, the compounds of the present invention do not act on or extremely weakly act on other receptors, channels, and enzymes. Therefore, it is expected that the compounds of the present invention do not show at all or extremely weakly show adverse effects such as convulsion, muscle relaxation, sedation, and cognitive function decline.

Since high blood concentration and enhanced migration into the brain of the compounds of the present invention may be achieved by oral administration or administration by injection (for example, subcutaneous injection), they can be used by oral administration or administration by injection.

Therefore, the compound of the present invention is a safe drug having high efficacy.

### Brief Description of the Drawings

Fig. 1 is a graph illustrating a result of a mouse elevated plus maze test for a compound described in Example 12.
Fig. 2 is a graph illustrating a result of a rat elevated plus maze test for the compound described in Example 12.

### Modes for Carrying out the Invention

Hereafter, the present invention will be explained in more detail.

Preferred embodiments of the compound represented by the general formula (I), a tautomer of the compound, stereoisomer of the compound, a pharmaceutically acceptable salt thereof, or a solvate thereof include the followings.
(1) A compound represented by the general formula (I), wherein R¹ represents hydrogen; C₁₋₁₀ alkyl; cycloalkylalkyl where the cycloalkyl moiety has 3 to 6 carbon atoms, and the alkylene moiety has 1 to 5 carbon atoms; aralkyl where the aryl moiety has 6 to 10 carbon atoms, and the alkylene moiety has 1 to 5 carbon atoms;, a tautomer of the compound, a stereoisomer of the compound, a pharmaceutically acceptable salt thereof, or a solvate thereof.
(2) A compound represented by the general formula (I), wherein R¹ is cycloalkylalkyl where the cycloalkyl moiety has 3 to 6 carbon atoms, and the alkylene moiety has 1 to 5 carbon atoms, a tautomer of the compound, a stereoisomer of the compound, a pharmaceutically acceptable salt thereof, or a solvate thereof.
(3) A compound represented by the general formula (I), wherein R¹ is C2-6 alkyl substituted with hydroxy; C₁₋₆ alkyl substituted with 1 to 6 halogens; or C₂₋₆ alkyl substituted with C₁₋₆ alkoxy, a tautomer of the compound, a stereoisomer of the compound, a pharmaceutically acceptable salt thereof, or a solvate thereof.
(4) A compound represented by the general formula (I), wherein R¹ is allyl, fluoropropyl, 2-(pyridyl-3-yl) ethyl, 2-(methylsulfonyl) ethyl, or 2-(aminosulfonyl) ethyl, a tautomer of the compound, a stereoisomer of the compound, a pharmaceutically acceptable salt thereof, or a solvate thereof.
(5) A compound represented by the general formula (I) or the compound according to (1) to (4), wherein R² represents a 5- to 7-membered saturated heterocycle containing one or two heteroatoms which may be the same or different and are selected from N and O and three or more carbon atoms as ring-constituting atoms, a tautomer of the compound, a stereoisomer of the compound, a pharmaceutically acceptable salt thereof, or a solvate thereof.
(6) A compound represented by the above general formula (I) or the compound according to (1) to (4), wherein R² is represented by the following general formula (II), 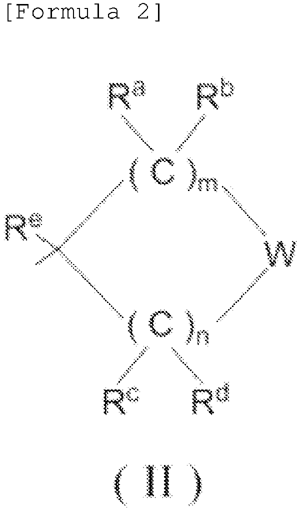
   (wherein, R^{a} to R^{e}, which are the same or different, represents hydrogen; hydroxy; halogen , C₁₋₆ alkyl, C₁₋₆ alkoxy, C₁₋₆ alkyl substituted with 1 to 3 halogens, or a C₁₋₆ alkoxy substituted with 1 to 3 halogens, or R^{a} and R^{b} or R^{c} and R^{d} combine together to represent an oxo group, wherein
   each of C(R^{a})(R^{b})s and each of C(R^{c})(R^{d})s may be the same or different, respectively, when there are a plurality of C(R^{a})(R^{b}) s and a plurality of C(R^{c})(R^{d})s,
   W represents O or NR^{f}, wherein R^{f} represents hydrogen; C₁₋₆ alkyl; C₁₋₆ alkyl substituted with 1 to 3 halogens; or alkoxycarbonyl where the alkoxy moiety has 1 to 6 carbon atoms, and
   m and n each represent 0 or an integer of 1 to 5, and m + n represents 3 to 5),
   a tautomer of the compound, a stereoisomer of the compound, a pharmaceutically acceptable salt thereof, or a solvate thereof.
(7) A compound represented by the general formula (I) or the compound according to (1) to (4), wherein R² represents pyrrolidinyl, piperidinyl, or tetrahydrofuranyl which may be substituted with 1 to 6 hydroxys which may be the same or different; halogen; C₁₋₆ alkyl; C₁₋₆ alkoxy; C₁₋₆ alkyl substituted with 1 to 3 halogens; C₁₋₆ alkoxy substituted with 1 to 3 halogens; or 1 or 2 oxo groups, a tautomer of the compound, a stereoisomer of the compound, a pharmaceutically acceptable salt thereof, or a solvate thereof.
(8) A compound represented by the general formula (I) or the compound according to (1) to (7), wherein X represents CH₂, a tautomer of the compound, a stereoisomer of the compound, a pharmaceutically acceptable salt thereof, or a solvate thereof.
(9) A compound represented by the general formula (I) or the compound according to (1) to (8), wherein Y represents C = O, a tautomer of the compound, a stereoisomer of the compound, a pharmaceutically acceptable salt thereof, or a solvate thereof.
(10) A compound represented by the general formula (I) or the compound according to (1) to (9), wherein one of R³ and R⁴ is hydroxy and the other is hydrogen, a tautomer of the compound, a stereoisomer of the compound, a pharmaceutically acceptable salt thereof, or a solvate thereof.
(11) A compound represented by the general formula (I) or the compound according to (1) to (9), wherein R³ represents halogen; cyano; carbamoyl; C₁₋₆ alkoxy, C₁₋₆ alkanoyloxy; amino, or acylamino where the acyl moiety has 2 to 6 carbon atoms, R⁴ represents hydrogen or hydroxy, and R⁵ represents hydrogen, a tautomer of the compound, a stereoisomer of the compound, a pharmaceutically acceptable salt thereof, or a solvate thereof.
(12) A compound represented by the general formula (I) or the compound according to (1) to (9), wherein R³ represents hydroxy; carbamoyl; or C₁₋₆ alkanoyloxy, R⁴ represents hydrogen, and R⁵ represents hydrogen, a tautomer of the compound, a stereoisomer of the compound, a pharmaceutically acceptable salt thereof, or a solvate thereof.
(13) A compound represented by the general formula (I) or the compound according to (1) to (9), wherein R³ represents hydroxy, R⁴ represents hydrogen, and R⁵ represents hydrogen, a tautomer of the compound, a stereoisomer of the compound, a pharmaceutically acceptable salt thereof, or a solvate thereof.
(14) A compound represented by the general formula (I) or the compound according to (1) to (9), wherein R³, R⁴, and R⁵ each represent hydrogen, a tautomer of the compound, a stereoisomer of the compound, a pharmaceutically acceptable salt thereof, or a solvate thereof.
(15) A compound represented by the general formula (I) or the compound according to (1) to (14), wherein R^{6a}, R^{6b}, R⁷, R⁸, R⁹, and R¹⁰ each represent hydrogen, a tautomer of the compound, a stereoisomer of the compound, a pharmaceutically acceptable salt thereof, or a solvate thereof.
(16) A compound selected from (1S,3aR,5aS,6R,11bR,11cS)-3-(L-prolyl)-14-(cyclopropylmethyl)-10-hydroxy-2,3,3a,4,5,6,7,11c-octahydro-1H-6,11b-(epiminoethano)-1,5a-methanonaphtho[1,2-e]indole, (1S,3aR,5aS,6R,11bR,11cS)-3-(D-prolyl)-14-(cyclopropylmethyl)-10-hydroxy-2,3,3a,4,5,6,7,11c-octahydro-1H-6,11b-(epiminoethano)-1,5a-methanonaphtho[1,2-e]indole, ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((S)-piperidin-2-yl) methanone,
   ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((R)-piperidin-2-yl) methanone,
   ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((R)-piperidin-3-yl) methanone,
   ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((S)-piperidin-3-yl) methanone,
   ((1S,3aR,5aS,6R,11bR,11cS)-14-(cyclopropylmethyl)-10-hydroxy-1,2,3a,4,5,6, 7,11c-octahydro-3H-6,11b-(epiminoethano)-1,5a-methanonaphtho[1,2-e]indol-3-yl) (piperidin-4-yl) methanone,
   4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl) piperidine-2,6-dione,
   (1S,3aR,5aS,6R,11bR,11cS)-14-(cyclopropylmethyl)-10-hydroxy-3-(methyl-D-prolyl)-2,3,3a,4,5,6,7,11c-octahydro-1H-6,12b-(epiminoethano)-1,5a-methanonaphtho[1,2-e]indol, (R)-5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl) pyrrolidin-2-one,
   (R)-5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyrrolidin-2-one,
   ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((R)-tetrahydrofuran-2-yl) methanone, and
   (R)-5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl) dihydrofuran-2(3H)-one,
   a tautomer of the compound, a stereoisomer of the compound, a pharmaceutically acceptable salt thereof, or a solvate thereof.

As used herein ,
examples of the C₁₋₆ alkyl include methyl, ethyl, propyl, i-propyl, butyl, tert-butyl, pentyl, neopentyl, and hexyl.

Examples of the C₁₋₁₀ alkyl include heptyl and octyl in addition to those exemplified above for the C₁₋₆ alkyl.

Examples of the C₁₋₆ alkyl substituted with 1 to 3 halogens include 2-chloroethyl, 2-fluoroethyl, 3-fluoropropyl, 2,2-difluoroethyl, trifluoromethyl, and 3,3,3-trifluoropropyl.

Examples of the C₂₋₆ alkenyl include 2-propenyl and 3-methyl-2-butenyl.

Examples of the cycloalkylalkyl where the cycloalkyl moiety has 3 to 6 carbon atoms, and the alkylene moiety has 1 to 5 carbon atoms include methyl, ethyl, and the like substituted with C3-6 cycloalkyl such as cyclopropyl, cyclobutyl, cyclopentyl, and cyclohexyl.

Examples of the aralkyl where the aryl moiety has 6 to 10 carbon atoms, and the alkylene moiety has 1 to 5 carbon atoms include benzyl group, and phenethyl group.

Examples of the C₃₋₆ cycloalkyl include cyclopropyl, cyclobutyl, cyclopentyl, and cyclohexyl.

Examples of the C₆₋₁₀ aryl include phenyl and naphthyl.

Examples of the heteroaryl containing 1 to 4 heteroatoms selected from N, O, and S as ring-constituting atoms include pyridyl, furyl, imidazolyl, pyrazolyl, pyrimidinyl, pyrazinyl, pyridazinyl, and thiazolyl.

The heteroaryl contains 1 to 4 heteroatoms selected from N, O, and S as ring-constituting atoms. Examples of the heteroarylalkyl where the heteroaryl moiety contains 1 to 4 heteroatoms selected from N, O, and S as ring-constituting atoms and the alkylene moiety has 1 to 5 carbon atoms include
(pyridin-2-yl) methyl, (pyridin-3-yl) methyl, (pyridin-4-yl) methyl, (furan-2-yl) methyl, (furan-3-yl) methyl, (imidazol-2-yl) methyl, (imidazol-4-yl) methyl, (imidazol-5-yl) methyl, (thiazol-2-yl) methyl, (thiazol-4-yl) methyl, (thiazol-5-yl) methyl, 2-(pyridin-2-yl) ethyl, 2-(pyridin-3-yl) ethyl, 2-(pyrazol-1-yl) ethyl, 2-(thiophen-2-yl) ethyl, and 2-(thiophen-3-yl) ethyl.

Examples of the C₁₋₆ alkanoyl include acetyl and propionyl.

Examples of the C₁₋₆ alkoxy include methoxy, ethoxy, and propoxy.

Examples of the C₁₋₆ alkanoyloxy include acetoxy.

Examples of the alkoxycarbonyl where the alkoxy moiety has 1 to 6 carbon atoms include methoxycarbonyl and ethoxycarbonyl.

Examples of the halogen include fluorine, chlorine, bromine, and iodine.

Examples of the C₁₋₆ alkoxy substituted with 1 to 3 halogens include fluoroethoxy and trifluoroethoxy.

Examples of the C₁₋₆ alkoxy substituted with 1 to 6 halogens include pentafluoroethoxy in addition to the above-described C₁₋₆ alkoxy substituted with 1 to 3 halogens.

Examples of the phenylalkyl having 1 to 3 carbon atoms in an alkyl include benzyl.

Examples of the C₆₋₁₀ aryloxy include phenoxy.

Examples of the C₁₋₈ alkylamino include methylamino and ethylamino.

Examples of the acylamino where the acyl moiety has 2 to 6 carbon atoms include acetylamino.

Examples of C₆₋₁₀ arylamino include phenylamino.

Examples of the alkylcarbamoyl where the alkyl moiety has 1 to 6 carbon atoms include ethylcarbamoyl.

Examples of the dialkylcarbamoyl where the alkyl moiety has 1 to 6 carbon atoms include diethylcarbamoyl.

Examples of the alkylsulfonyl where the alkyl moiety has 1 to 6 carbon atoms include methylsulfonyl.

Examples of the alkylsulfinyl where the alkyl moiety has 1 to 6 carbon atoms include methylsulfinyl.

Examples of the alkylthio where the alkyl moiety has 1 to 6 carbon atoms include methylthio.

Examples of the arylcarbonyl where the aryl moiety has 6 to 10 carbon atoms include benzoyl.

Examples of the 5- to 7-membered ring which may be formed by R¹², R¹³, the nitrogen atom to which R¹² and R¹³ are bonded, and furthermore if desired, 1 to 2 heteroatoms together, include pyrrolidine, piperidine, and morpholine.

Examples of R² include pyrrolidine, piperidine, tetrahydrofuran, pyrrolidin-2-one, piperidine-2,6-dione, dihydrofuran-2-one, a N-C₁₋₆ alkylpyrrolidine, a N-C₁₋₆ alkylpiperidine, and a N-C₁₋₆ alkylpyrrolidin-2-one.

As for the compound represented by the general formula (I), a tautomer of the compound, a stereoisomer of the compound, a pharmaceutically acceptable salt thereof, or a solvate thereof, preferred examples of the pharmaceutically acceptable salt include an acid addition salt. Examples of the acid addition salt include a salt with an organic acid or an inorganic acid, such as a hydrochloride, a sulfate, a fumarate, an oxalate, a methanesulfonate, or a camphorsulfonate.

As for the compound represented by the general formula (I), a tautomer of the compound, a stereoisomer of the compound, a pharmaceutically acceptable salt thereof, or a solvate thereof, examples of the stereoisomer include a cis-isomer, a trans-isomer, a racemate, and an optically active substance.

As for the compound represented by the general formula (I), a tautomer or stereoisomer of the compound, or a pharmaceutically acceptable salt thereof, or a solvate thereof, the solvate is a pharmaceutically acceptable solvate of the compound of the present invention or a salt thereof, and includes hydrate.

The compound represented by the general formula (I), a tautomer or stereoisomer of the compound, or a pharmaceutically acceptable salt thereof, or a solvate thereof may be chemically modified into such a prodrug that it is converted into a pharmacologically active substance and exhibits the pharmacological activity (being activated) after it is delivered into the inside of the body or a target site.

Furthermore, a compound represented by the general formula (I), a tautomer of the compound, a stereoisomer of the compound, a pharmaceutically acceptable salt thereof, or a solvate thereof may be substituted with an atom of a stable isotope such as deuterium.

Hereafter, methods for preparing the compound represented by the general formula (I), a tautomer of the compound, a stereoisomer of the compound, a pharmaceutically acceptable salt thereof, or a solvate thereof will be shown below.

The abbreviations used here are as follows.

### Abbreviation table

Boc: tert-butoxycarbonyl
CPM: cyclopropylmethyl
DMA: N,N-dimethylacetamide
DMAP: N,N-dimethyl-4-aminopyridine
DMF: N,N-dimethylformamide
HATU: 1-[bis(dimethylamino) methylene]-1H-1,2,3-triazolo[4,5-b]pyridinium 3-oxide hexafluorophosphate
HOBT: 1-hydroxybenzotriazole
Me: methyl
Ms: mesyl
Ph: phenyl
TBS: tert-butyldimethylsilyl
THF: tetrahydrofuran
TLC: thin layer chromatography
Ts: tosyl
WSC: 1-ethyl-3-(3-dimethylaminopropyl)carbodiimide hydrochloride

### (Preparation method)

### Compound provided by the present invention represented by the general formula (I), wherein Y represents C = O and R⁵, R^{6a}, R^{6b}, R⁷, R⁸, R⁹, and R¹⁰ each represent hydrogen

The following compound (I) which is the compound provided by the present invention can be obtained, for example, by a deprotection reaction for converting the following compound (I-A) into the compound (I).

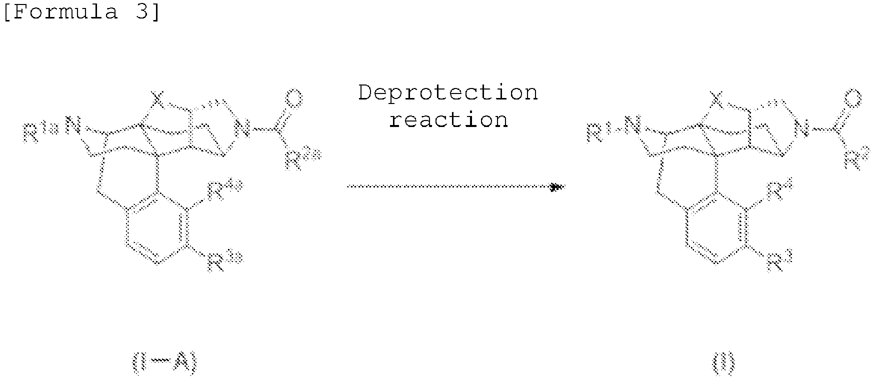

[In the formulas, R^{1a}, R^{2a}, R^{3a}, and R^{4a} are arbitrary functional groups that can be converted into R¹, R², R³, and R⁴ in the general formula (I), respectively, or R^{1a} itself may be R¹, R^{2a} itself may be R², R^{3a} itself may be R³, and R^{4a} itself may be R⁴. The other symbols have the same meanings as those defined above.]

In the aforementioned preparation method, the aforementioned compound (I) can be prepared by performing an appropriate known general deprotection reaction as required to convert R1a of the aforementioned compound (I-A) into R¹, R^{2a} of the same into R², R^{3a} of the same into R³, or R^{4a} of the same into R⁴. For example, when R^{1a}, R^{2a}, R^{3a}, or R^{4a} in the aforementioned compound (I-A) contains hydroxy group protected with methyl group, the methyl group as the protective group can be removed by (1) a method of allowing boron tribromide to act on the aforementioned compound (I-A) in dichloromethane, or (2) a method of heating the aforementioned compound (I-A) together with a large excess amount of pyridine hydrochloride in the absence of solvent, and thereby, the aforementioned compound (I) can be prepared.

When R^{1a}, R^{2a}, R^{3a}, or R^{4a} in the aforementioned compound (I-A) contains hydroxy group protected with tert-butyldimethylsilyl (TBS) group, the TBS group as the protective group can be removed by (3) a method of allowing ammonia dissolved in an appropriate solvent to act on the aforementioned compound (I-A), (4) a method of allowing hydrogen chloride dissolved in an appropriate solvent to act on the aforementioned compound (I-A), or (5) a method of allowing tetrabutylammonium fluoride to act on the aforementioned compound (I-A) in THF, or the like, and thereby, the aforementioned compound (I) can be prepared.

When R^{1a}, R^{2a}, R^{3a}, or R^{4a} contains a functional group protected with another protective group, the aforementioned compound (I) can be prepared from the aforementioned compound (I-A) under the general deprotection conditions such as those explained in Peter G.M. Wuts, "Green's Protective Groups in Organic Synthesis (5th edition, A John Wiley & Son's, Inc., Publication).

When R^{1a}, R^{2a}, R^{3a}, and R^{4a} have different protective groups, and they must be removed under different conditions, deprotection reactions may be successively performed under different conditions suitable for removing the protective groups as a multi-step deprotection reaction to prepare the aforementioned compound (I) from the aforementioned compound (I-A).

The aforementioned compound (I-A) can be obtained by, for example, performing a general acylation reaction for the following compound (I-B) mentioned in the reaction formula shown below.

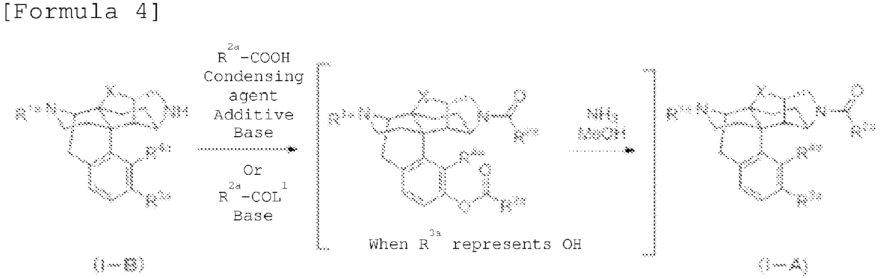

[In the formulas, R^{1a}, R^{2a}, R^{3a}, and R^{4a} are arbitrary functional groups that can be converted into R¹, R², R³, and R⁴ in the general formula (I), respectively, or R^{1a} itself may be R¹, R^{2a} itself may be R², R^{3a} itself may be R³, and R^{4a} itself may be R⁴. L¹ represents a leaving group of a common acylating agent. The other symbols have the same meanings as those defined above.]

In the aforementioned preparation method, the aforementioned compound (I-A) can be obtained by reacting the aforementioned compound (I-B), a carboxylic acid (R2aCOOH), and a condensing agent such as HATU and WSC in the presence of an additive such as HOBT and DMAP, and a base such as triethylamine and diisopropylethylamine, as required.

The aforementioned compound (I-A) can also be obtained by reacting the aforementioned compound (I-B), a carboxylic acid chloride (R^{2a}COCl, L¹ in the formula = Cl) or a carboxylic anhydride (L¹ in the formula = -OC(O)R^{2a}) in the presence of a base such as triethylamine, diisopropylethylamine, and pyridine.

When R^{3a} is hydroxy group (OH), in the acylation reaction mentioned in the above reaction formula, acylation of hydroxy group of R^{3a} also progresses as a side reaction in addition to the desired amidation reaction, and a product corresponding to the aforementioned compound (I-A) wherein R^{3a} = -OC(O)R^{2a} is temporarily obtained as a byproduct in the reaction system. However, by treating the reaction solution with a 2 N ammonia solution in methanol or the like, such a compound is converted again into a compound where R3a = OH in a post-treatment process, and the aforementioned compound (I-A) resulting from selective amidation of the secondary amine in the aforementioned compound (I-B) can be obtained as a result.

In addition, the aforementioned compound (I-A) can also be synthesized from the aforementioned compound (I-B) and a corresponding carboxylic acid (R^{2a}-COOH) according to the condensation reaction explained in Christian A.G.N. Montalbetti, et al., Tetrahedron, 61(46), 2005, 10827-10852.

A desired compound (I-A) can be obtained by using, for example, the compounds described in WO2013/035833 such as compound 8 (Example 4, R^{1a} = CPM, X = O, R^{3a} = OMe, R^{4a} = H), compound 33 (Example 29, R^{1a} = Me, X = O, R^{3a} = OMe, R^{4a} = H), compound 67 (Example 60, R^{1a} = CPM, X = O, R^{3a} = H, R^{4a} = OH), compound 77 (Example 67, R^{1a} = CPM, X = CH2, R^{3a} = OMe, R^{4a} = H), compound 116 (Example 101, R^{1a} = CPM, X = CH₂, R^{3a} = H, R^{4a} = OH), compound 130 (Example 106, R^{1a} = PhCF2CH2, X = CH₂, R^{3a} = OMe, R^{4a} = H), compound 185 (Example 143, R^{1a} = TBSOCH2CH2, X = CH2, R^{3a} = OMe, R^{4a} = H), compound 189 (Example 144, R^{1a} = (R)-MeCH(OH)CH2, X = CH2, R^{3a} = OMe, R^{4a} = H), compound 350 (Example 261, R^{1a} = (S)-MeCH(OH)CH₂, X = CH₂, R^{3a} = OMe, R^{4a} = H), compound 291 (Example 224, R^{1a} = CPM, X = CH₂, R^{3a} = H, R^{4a} = OMe), and compound 297 (Example 228, R^{1a} = CPM, X = CH₂, R^{3a} = H, R^{4a} = H), and the compounds described in WO2014/136305 such as compound 29 (Example 27, R^{1a} = BocNHCH2CH2, X = CH2, R^{3a} = OTBS, R^{4a} = H), compound 35 (Example 19, R^{1a} = CF₃CO, X = CH2, R^{3a} = OMe, R^{4a} = H), and compound 68 (Example 34, R^{1a} = Boc, X = CH2, R^{3a} = OMe, R^{4a} = H) as the aforementioned compound (I-B), or by a combination of a known conversion of functional group and deprotection reaction performed by a method described in the aforementioned patent documents.

The following compound (I-A) can also be obtained by, for example, a common alkylation reaction of the following compound (I-C) mentioned in the reaction formula shown below.

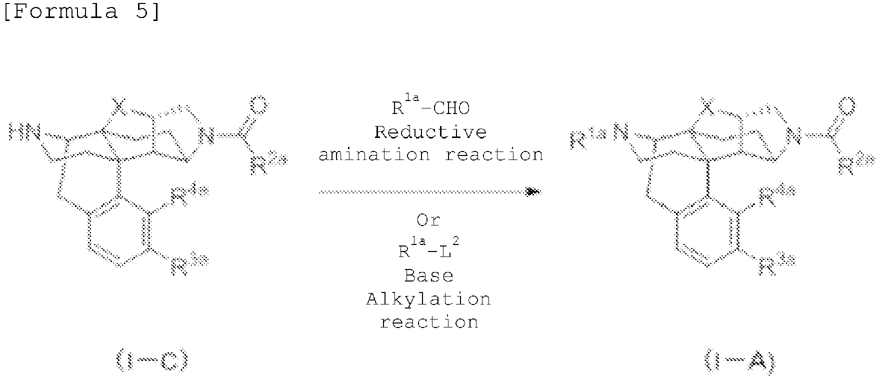

[In the formulas, R^{1a}, R^{2a}, R^{3a}, and R^{4a} are arbitrary functional groups that can be converted into R¹, R², R³, and R⁴ in the general formula (I), respectively, or R1a itself may be R¹, R^{2a} itself may be R², R^{3a} itself may be R³, and R^{4a} itself may be R⁴. L² represents a leaving group for a common alkylating reaction, R^{1'a} represents such a substituent that R^{1'a}-CH2 = R1a is satisfied, and the other symbols have the same meanings as those defined above.]

In the aforementioned preparation method, the aforementioned compound (I-A) can be synthesized by allowing a corresponding aldehyde (R^{1'a}-CHO, R^{1'a} represents such a substituent that R^{1'a}-CH2 = R^{1a} is satisfied), and a reducing agent such as sodium triacetoxyborohydride or sodium cyanoborohydride to act on the aforementioned compound (I-C) in an appropriate solvent in the presence of an additive such as acetic acid as required.

The aforementioned compound (I-A) can also be synthesized by allowing a corresponding alkylating agent (R1a-L2, L2 represents an appropriate leaving group, for example, halogen such as Cl, Br, and I, OMs, or OTs) to act on the aforementioned compound (I-C) in a polar solvent such as DMF or an alcohol in the presence of a base such as potassium carbonate.

In addition, the method for introducing the R1a group into the aforementioned compound (I-C) is not limited to the reactions described above, and by using a known general alkyl group introduction reaction for amino group, which may be a multi-step reaction, the aforementioned compound (I-A) can be prepared from the aforementioned compound (I-C).

The aforementioned compound (I-C) can be synthesized by a combination of known functional group conversion and deprotection reaction of an appropriate starting material described in any of the aforementioned references according to a method similar to any of the synthesis methods of, for example, the compounds described in WO2013/035833 such as compound 11 (Example 7, R^{2a} = Ph, X = O, R^{3a} = OMe, R^{4a} = H), compound 81 (Example 71, R^{2a} = Ph, X = CH2, R^{3a} = OMe, R^{4a} = H), compound 121 (Example 104, R^{2a} = Ph, X = CH₂, R^{3a} = OTBS, R^{4a} = H), compound 149 (Example 120, R^{2a} = 2-pyridil, X = CH₂, R^{3a} = OMe, R^{4a} = H), compound 116 (Example 101, R^{1a} = CPM, X = CH2, R^{3a} = OMe, R^{4a} = H), and compound 217 (Example 163, R^{2a} = CF3, X = CH₂, R^{3a} = OMe, R^{4a} = H) . Another compound represented by the general formula (I) as the compound of the present invention can be prepared by combining the above preparation method, a method described in Examples below, the above Patent Literatures, the above Non Patent Literatures, and the like.

The compound represented by the general formula (I), a tautomer or stereoisomer thereof, or a pharmaceutically acceptable salt or a solvate thereof showed superior agonistic activity and selectivity for the opioid δ receptor in a test concerning functional activities for the µ, δ, and κ opioid receptors (see Table 1 in Example 15).

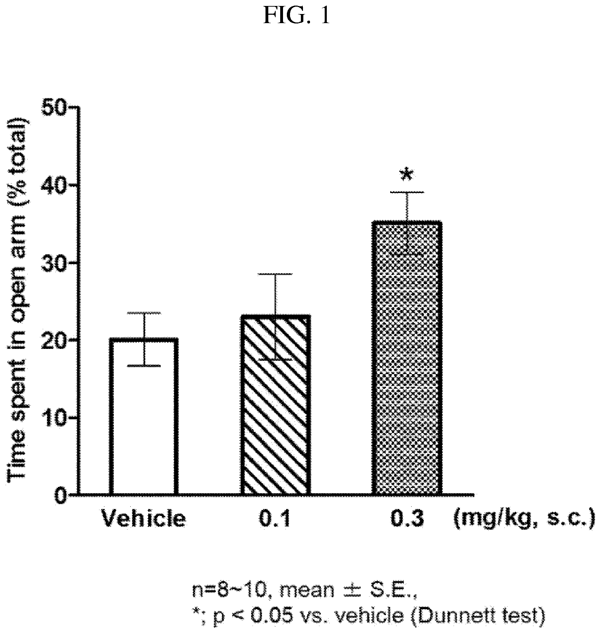

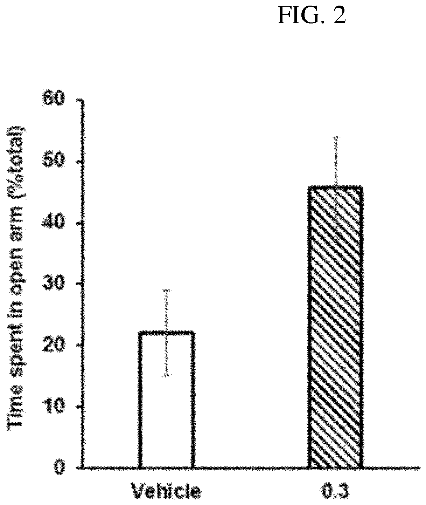

In addition, the compound represented by the general formula (I), a tautomer or stereoisomer thereof, or a pharmaceutically acceptable salt or a solvate thereof significantly increased the wall-less running route (open arm) staying time ratio in the mouse and rat elevated plus maze tests, and thus exhibited anxiolytic-like effects (see Figs. 1 and 2 of Examples 16 and 17). The elevated plus maze tests were performed according to the method described in Non-patent document 6.

Furthermore, the compound represented by the general formula (I), a tautomer or stereoisomer thereof, or a pharmaceutically acceptable salt or a solvate thereof showed only weak inhibitory action in a hERG (human ether-a-go-go-related gene) potassium channel inhibition test as described in Example 18 mentioned later. This indicates that the risks of the compounds represented by the general formula (I), tautomers or stereoisomers of the compounds, pharmaceutically acceptable salts thereof, and solvates thereof for retarding the ventricular repolarization and prolonging the QT interval in humans are low.

In addition, it has been revealed that a compound represented by the general formula (I), a tautomer of the compound, a stereoisomer of the compound, a pharmaceutically acceptable salt thereof, or a solvate thereof exhibits a sufficient central transportation property due to exhibiting a medicinal effect (see Example 19), is capable of oral administration, and has an anxiolytic effect.

Therefore, in consideration of the above-described Patent Literatures, Non Patent Literatures, and the like, a compound represented by the general formula (I), a tautomer of the compound, a stereoisomer of the compound, a pharmaceutically acceptable salt thereof, or a solvate thereof can be used for the treatment and the prevention of depression or anxiety, and can be used as prophylactic and therapeutic agents for psychiatric disorders included in the depression disorder group, anxiety disorder group, bipolar disorder group, obsessive-compulsive disorder and related disorder group, psychic trauma and stress factor-related disorder group, and the like described in DSM-5 (Diagnostic and Statistical Manual of Mental Disorders, 5th edition, American Psychiatric Association) (antidepressants, anxiolytic drugs, etc.), and as prophylactic and therapeutic agents for neurodegenerative diseases such as urinary incontinence, myocardial ischemia, brain ischemia, chronic coughing, hypertension, Parkinson's disease, and epilepsy.

As used herein, depression may be a state that there is observed a combination of a mood disorder such as depressive feeling, sad feeling, and lonely feeling, decrease in activity volition, congestion of ideas, pessimistic idea, and an autonomic nerve disorder such as sleep disorder and decrease in appetite. As used herein, anxiety may be a state of feeling danger or fear with restlessness, strain, tachycardia, breathing difficulty, and the like, although the state is not connected with a stimulus that can be clearly confirmed. The depression and anxiety include the depressive and anxiety symptoms observed in the psychiatric disorders described in DSM-5 mentioned above (for example, depressive symptoms observed in bipolar disorders, and depressive and anxious symptoms observed in PTSD), depressive state of which symptoms are milder than those of the depression disorders described in DSM-5, but are maintained in a certain degree, and anxious state of which symptoms are milder than those of the anxiety disorders described in DSM-5, but are maintained in a certain degree.

Further, the compounds represented by the general formula (I), tautomers or stereoisomers of the compounds, or pharmaceutically acceptable salts thereof, or solvates thereof may be used as medicaments for assisting the therapeutic treatment of any of the aforementioned diseases.

The compounds represented by the general formula (I), tautomers or stereoisomers of the compounds, pharmaceutically acceptable salts thereof, and solvates thereof can also be used for therapies of pains regarding diseases accompanied by an acute pain or chronic pain, or as prophylactic and therapeutic agents for pains of rheumatoid arthritis, osteoarthritis deformans, cancer pain accompanied by severe pain such as osteoncus, diabetic neuropathic pain, postherpetic neuralgia, visceral pains, and the like.

The compounds represented by the general formula (I), tautomer or stereoisomers of the compounds, pharmaceutically acceptable salts thereof, and solvates thereof are preferably expected to be antidepressants and anxiolytic drugs. The compounds represented by the general formula (I), tautomers or stereoisomers of the compounds, pharmaceutically acceptable salts thereof, and solvates thereof can be administered to a human by an appropriate administration method such as oral administration or parenteral administration. Further, they can be used together with other anxiolytic drugs, antidepressants, and analgesics.

As for preparation of pharmaceutical preparations thereof, they can be prepared in a dosage form of tablet, granule, powder, capsule, suspension, injection, suppository or the like by methods commonly used in the field of pharmaceuticals.

For preparation of such pharmaceutical preparations, for example, commonly used excipients, disintegrating agents, binders, lubricants, dyes, and the like are used in the case of tablet. Examples of the excipients include lactose, D-mannitol, crystalline cellulose, glucose, and the like. Examples of the disintegrating agents include starch, carboxymethylcellulose calcium (CMC-Ca), and the like. Examples of the lubricants include magnesium stearate, talc, and the like. Examples of the binders include hydroxypropylcellulose (HPC), gelatin, polyvinylpyrrolidone (PVP), and the like. For the preparation of injection, solvents, stabilizers, dissolving aids, suspending agents, emulsifiers, soothing agents, buffering agents, preservatives, and the like are used.

As for the dose of the compound represented by the aforementioned general formula (I), a tautomer or stereoisomer of the compound, or a pharmaceutically acceptable salt thereof, or a solvate thereof as active ingredient, it is usually administered to adults at a dose of 0.1 µg to 1 g/day, preferably 0.001 to 200 mg/day, in the case of injection; or at a dose of 1 µg to 10 g/day, preferably 0.01 to 2000 mg/day, in the case of oral administration, but the dose may be decreased or increased depending on age, symptoms, and the like.

Hereafter, the present invention will be further explained in more detail with reference to reference examples and examples. However, the present invention is not limited to these examples.

Names of the compounds mentioned in the examples and reference examples are obtained by converting structural formulas depicted with ChemDraw ver. 14, Cambridge Software into English compound names with a naming algorithm of the same software, and translating them into Japanese names. Examples

### Reference Example 1

### Synthesis of (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indol-10-ol

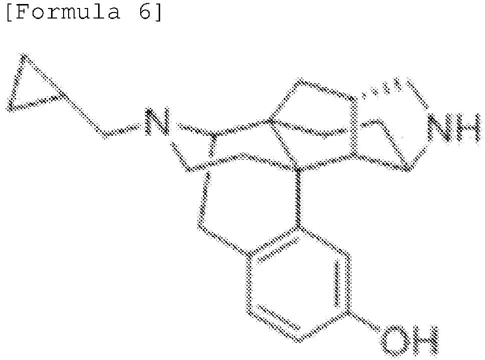

Into a 300-mL round bottom flask, (1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphth[1,2-e]indole (372 mg, 1.02 mmol) synthesized according to the method of WO2013/035833, Example 67 was added, and dissolved in dichloromethane (5 mL), the solution was vigorously stirred at 0°C for 20 minutes, then a 1.0 M solution of boron tribromide in dichloromethane (5 mL, 5 mmol) was added to the solution, and the resulting mixture was stirred at room temperature for 30 minutes. To the reaction solution, methanol (10 mL) was added at 0°C, and the resulting mixture was stirred at the same temperature for 1 hour.

The reaction solution was concentrated under reduced pressure, and the residue was suspended in chloroform (50 mL), and washed with 6% aqueous ammonia (20 mL). The aqueous layer was extracted twice with chloroform (30 mL), the combined organic layers were dried over anhydrous sodium sulfate, the insoluble matter was separated by filtration, and then the filtrate was concentrated under reduced pressure to obtain the title compound (356 mg, 100%) as brown foam. [Alternative method]

Into a 500-mL round bottom flask, (1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphth[1,2-e]indole (3.58 g, 9.82 mmol) synthesized according to the method of WO2013/035833, Example 67, and pyridine hydrochloride (87 g, 753 mmol) were added, and the mixture was stirred at 200°C for 1 hour. After the reaction, the reaction mixture was returned to room temperature, saturated aqueous potassium carbonate was added to the produced solid to dissolve it, the solution was extracted with ethyl acetate and chloroform, and the combined organic layers were dried over anhydrous sodium sulfate. The insoluble matter was separated by filtration, and then the filtrate was concentrated under reduced pressure to obtain the title compound (3.30 g, 96%) as brown foam.
¹H NMR (CDCl₃, 400MHz) : δ = 6.94 (d, 1H, J = 8.2Hz), 6.70 (dd, 1H, J = 8.2Hz, 2.8Hz), 6.50 (d, 1H, J = 2.3Hz), 3.73-3.76 (m, 1H), 3.23-3.31 (m, 2H), 3.05-3.12 (m, 2H), 2.77-2.99 (m, 4H), 2.55 (dd, 1H, J = 11.0Hz, 5.0Hz), 2.31 (d, 1H, J = 6.4Hz), 1.91-2.11 (m, 2H), 1.69-1.74 (m, 1H), 1.20-1.45 (m, 3H), 0.93-1.10 (m, 3H), 0.77-0.83 (m, 1H), 0.42-0.51 (m, 2H), 0.05-0.14 (m, 2H). NH and OH are not seen.

### Reference Example 2

### Synthesis of (tert-butoxycarbonyl)-L-proline

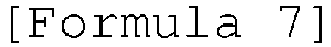

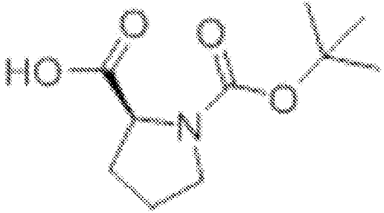

Into a 50 mL round bottom flask, L-Proline (500 mg, 4.3 mmol) and a saturated sodium bicarbonate aqueous solution (6.6 mL) were added, and di-tert-butyl dicarbonate (1 mL, 4.8 mmol) dissolved in THF (5 mL) was added dropwise thereto in an ice bath. Thereafter, the resulting mixture was stirred at room temperature for 19 hours. THF was distilled off. Thereafter, ethyl acetate was added, and 3 N hydrochloric acid was added dropwise until the aqueous layer reached pH 2. Extraction with ethyl acetate was performed for the reaction solution three times, and the collected organic layer was dried over sodium sulfate. The insoluble matter was distilled off. Thereafter, the filtrate was concentrated under reduced pressure to obtain the title compound.
¹H NMR (CDCl₃, 400MHz) : δ = 4.21-4.27 (m, 1H), 3.26-3.64 (m, 2H), 2.20-2.52 (m, 1H), 1.81-2.14 (m, 3H), 1.47 (br s, 6H), 1.43 (br s, 3H). CO₂H is not seen.

### Reference Example 3

### Synthesis of (tert-butoxycarbonyl)-D-proline

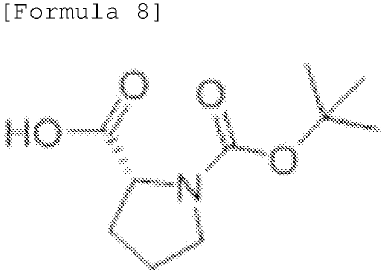

In a similar manner to Reference Example 2, the title compound was obtained using D-proline (50 mg, 0.43 mmol) and di-tert-butyl dicarbonate (0.11 mL, 0.48 mmol).

### Reference Example 4

### Synthesis of (S)-1-(tert-butoxycarbonyl) piperidine-2-carboxylic acid

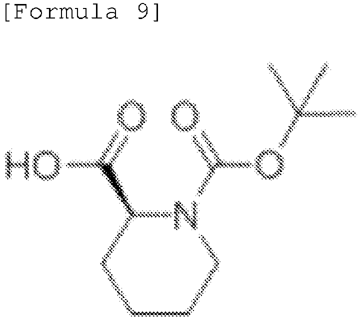

In a similar manner to Reference Example 2, the title compound was obtained using (S)-piperidine-2-carboxylic acid (50 mg, 0.39 mmol) and di-tert-butyl dicarbonate (98 µL, 0.43 mmol).
¹H NMR (CDCl₃, 400MHz) : δ = 9.92 (br s, 1H), 4.77-4.95 (m, 1H), 3.91-4.04 (m, 1H), 2.87-3.02 (m, 1H), 2.20-2.27 (m, 1H), 1.62-1.72 (m, 3H), 1.25-1.48 (m, 11H).

### Reference Example 5

### Synthesis of (R)-1-(tert-butoxycarbonyl) piperidine-2-carboxylic acid

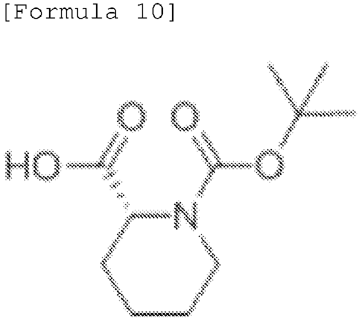

In a similar manner to Reference Example 2, the title compound was obtained using (R)-piperidine-2-carboxylic acid (75 mg, 0.58 mmol) and di-tert-butyl dicarbonate (0.15 mL, 0.64 mmol).
¹H NMR (CDCl₃, 400MHz) : δ = 10.05 (br s, 1H), 4.77-4.95 (m, 1H), 3.91-4.04 (m, 1H), 2.87-3.02 (m, 1H), 2.20-2.28 (m, 1H), 1.62-1.72 (m, 3H), 1.25-1.48 (m, 11H).

### Reference example 6

### Synthesis of (R)-1-(tert-butoxycarbonyl) piperidine-3-carboxylic acid

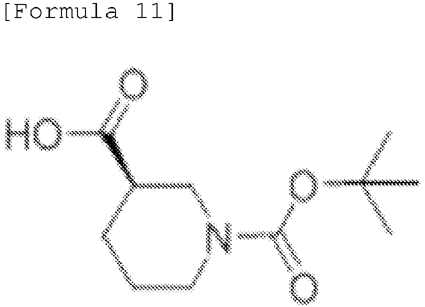

In a similar manner to Reference Example 2, the title compound was obtained using (R)-piperidine-3-carboxylic acid (75 mg, 0.58 mmol) and di-tert-butyl dicarbonate (0.15 mL, 0.64 mmol).
¹H NMR (CDCl₃, 400MHz) : δ = 10.22 (br s, 1H), 3.79-4.40 (m, 2H), 2.74-3.34 (m, 2H), 2.44-2.52 (m, 1H), 2.05-2.10 (m, 1H), 1.60-1.75 (m, 2H), 1.38-1.52 (m, 10H).

### Reference Example 7

### Synthesis of (S)-1-(tert-butoxycarbonyl) piperidine-3-carboxylic acid

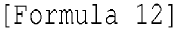

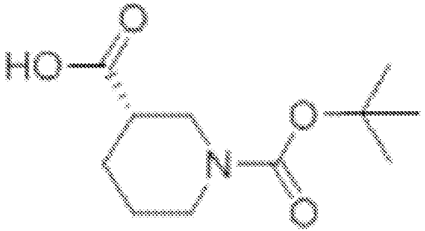

In a similar manner to Reference Example 2, the title compound was obtained using (S)-piperidine-3-carboxylic acid (75 mg, 0.58 mmol) and di-tert-butyl dicarbonate (0.15 mL, 0.64 mmol).
¹H NMR (CDCl₃, 400MHz) : δ = 10.28 (br s, 1H), 3.80-4.40 (m, 2H), 2.73-3.31 (m, 2H), 2.45-2.52 (m, 1H), 2.05-2.10 (m, 1H), 1.60-1.75 (m, 2H), 1.42-1.52 (m, 10H).

### Reference Example 8

### Synthesis of 1-(tert-butoxycarbonyl) piperidine-4-carboxylic acid

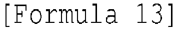

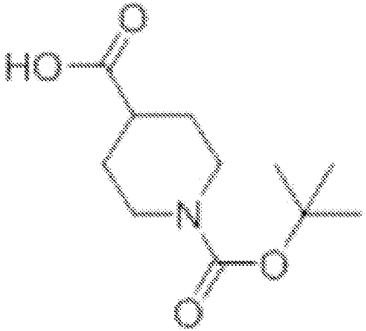

In a similar manner to Reference Example 2, the title compound was obtained using piperidine-4-carboxylic acid (50 mg, 0.39 mmol) and di-tert-butyl dicarbonate (98 µL, 0.43 mmol).
¹H NMR (CDCl₃, 400MHz) : δ = 10.00 (br s, 1H), 4.02 (br s, 2H), 2.83-2.89 (m, 2H), 2.45-2.53 (m, 1H), 1.89-1.93 (m, 2H), 1.59-1.69 (m, 2H), 1.46 (s, 9H).

### Reference Example 9

### Synthesis of methyl-D-proline

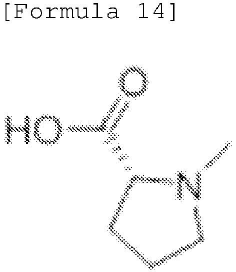

Into a 50 mL round bottom flask, D-proline (200 mg, 1.7 mmol), methanol (2 mL), a 37% formaldehyde aqueous solution (200 µL), and palladium carbon (50 mg) were added sequentially, and the resulting mixture was stirred at room temperature for 22 hours under a hydrogen atmosphere. The reaction solution was filtered through celite and the filtrate was concentrated under reduced pressure to obtain the title compound.
¹H NMR (DMSO-d₆, 400MHz) : δ = 3.38-3.47 (m, 2H), 2.80-2.87 (m, 1H), 2.68 (s, 3H), 2.13-2.23 (m, 1H), 1.84-1.94 (m, 2H), 1.64 - 1.77 (m, 1H). CO₂H is not seen.

### Reference Example 10

### Synthesis of (R)-1-methyl-5-oxopyrrolidine-2-carboxylic acid

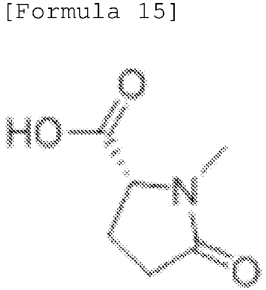

Into a 50 mL round bottom flask, DMF (5 mL), 60% dispersion sodium hydride (200 mg, 5.0 mmol), and (R)-5-oxopyrrolidine-2-carboxylic acid (258 mg, 2.0 mmol) were added, and the resulting mixture was stirred at room temperature for two hours. Methyl iodide (311 µL, 5.0 mmol) was added to the reaction mixture in an ice bath, and the resulting mixture was stirred at room temperature for 16 hours. Thereafter, a saturated sodium bicarbonate aqueous solution was added thereto, followed by extraction with ethyl acetate three times. The collected organic layer was dried over sodium sulfate. The insoluble matter was distilled off. Thereafter, the filtrate was concentrated under reduced pressure.

The obtained crude product was dissolved in methanol (1 mL) and water (0.1 mL), and hydroxylated lithium monohydrate (37 mg, 0.89 mmol) was added thereto. The resulting mixture was stirred at room temperature for three hours. To the reaction mixture, 3 N hydrochloric acid was added to make the aqueous layer acidic. Thereafter, the aqueous layer was washed with ethyl acetate. The aqueous layer was concentrated, and then methanol was added thereto. The resulting insoluble matter was filtered off, and concentration was performed to obtain the title compound.
¹H NMR (CD₃OD, 400MHz) : δ = 4.15-4.25 (m, 1H), 2.83 (s, 3H), 2.28-2.46 (m, 3H), 2.05-2.13 (m, 1H). CO₂H is not seen.

### Reference Example 11

### Synthesis of 2,2,2-trifluoro-1-((1S,3aR,5aS,6R,11bR,11cS)-10-hydroxy-2,3,3a,4,5,6,7,11c-octahydro-1H-6,11b-(epiminoethano)-1,5a-methanonaphtho[1,2-e]indol-14-yl) ethan-1-one

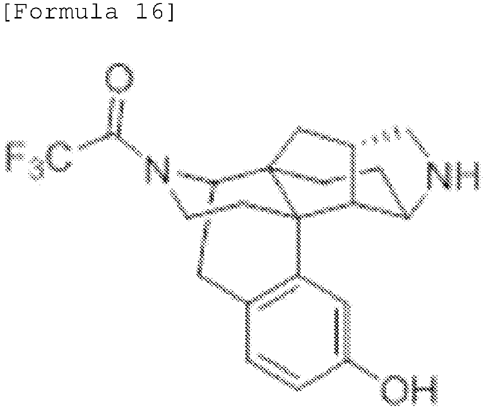

### (1) (1S,3aR,5aS,6R,11bR,11cS)-10-hydroxy-1,2,3a,4,5,6,7,11c-octahydro-3H-6,11b-(epiminoethano)-1,5a-methanonaphtho[1,2-e]indole-3-carboxylic acid 2,2,2-trichloroethyl

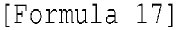

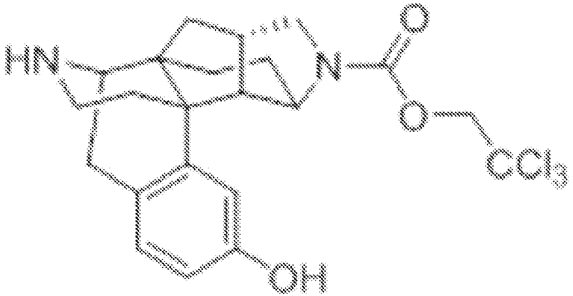

Into a 100 mL eggplant-shaped flask, (1S,3aR,5aS,6R,11bR,11cS)-10-methoxy-1,2,3a,4,5,6,7,11c-octahydro-3H-6,11b-(epiminoethano)-1,5a-methanonaphtho[1,2-e]indole-3-carboxylic acid 2,2,2-trichloroethyl synthesized by a method described in Example 34(1) of WO 2014136305 (972.7 mg, 2.00 mmol) was added and dissolved in methylene chloride (20 mL). The reaction solution was cooled to 0°C, and then a 1 M boron tribromide/methylenehe chloride solution (6 mL) was added thereto while being vigorously stirred. Thereafter, the resulting mixture was stirred for one hour while the temperature was raised to room temperature. A saturated sodium bicarbonate aqueous solution (30 mL) was added to the reaction solution, followed by extraction with chloroform (20 mL × 3). The collected organic layer was dried over anhydrous sodium sulfate. Thereafter, the insoluble matter was filtered off, and the filtrate was concentrated under reduced pressure to obtain the title compound as a white foam-like substance. The crude product was used as it was in the next reaction without further purification.

### (2) (1S,3aR,5aS,6R,11bR,11cS)-10-hydroxy-14-(2,2,2-trifluoroacetyl)-1,2,3a,4,5,6,7,11c-octahydro-3H-6,11b-(epiminoethano)-1,5a-methanonaphtho[1,2-e]indole-3-carboxylic acid 2,2,2-trichloroethyl

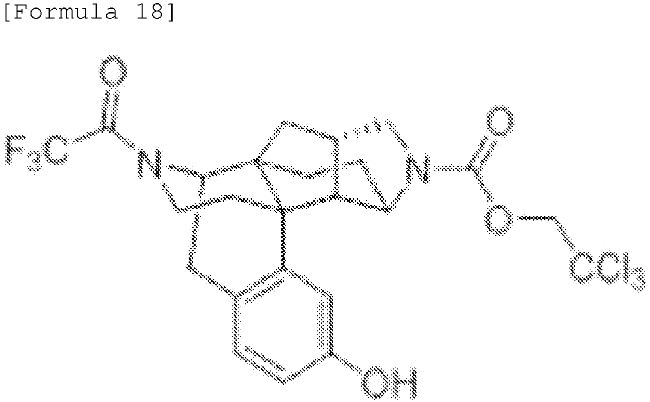

Into a 100 mL eggplant-shaped flask, (1S,3aR,5aS,6R,11bR,11cS)-10-hydroxy-1,2,3a,4,5,6,7,11c-octahydro-3H-6,11b-(epiminoethano)-1,5a-methanonaphtho[1,2-e]indole-3-carboxylic acid 2,2,2-trichloroethyl (1.04 g) obtained above was added and dissolved in THF (20 mL). Triethylamine (2.79 mL, 20 mmol) and trifluoroacetic anhydride (1.41 mL, 10 mmol) were added to the obtained solution, and the resulting mixture was stirred at room temperature for one hour. The reaction solution was concentrated under reduced pressure. The residue was diluted with a saturated sodium bicarbonate aqueous solution (50 mL), followed by extraction with ethyl acetate (30 mL x 2). The collected organic layer was dried over anhydrous sodium sulfate. Thereafter, the insoluble matter was filtered off, and the filtrate was concentrated under reduced pressure to obtain the title compound as a white foam-like substance. The crude product was used as it was in the next reaction without further purification.

### (3) 2,2,2-trifluoro-1-((1S,3aR,5aS,6R,11bR,11cS)-10-hydroxy-2,3,3a,4,5,6,7,11c-octahydro-1H-6,11b-(epiminoethano)-1,5a-methanonaphtho[1,2-e]indol-14-yl) ethan-1-one

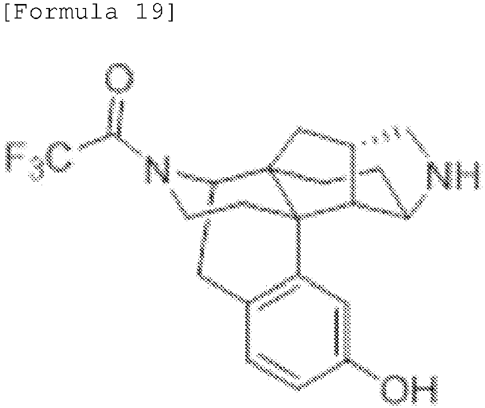

Into a 100 mL eggplant-shaped flask, (1S,3aR,5aS,6R,11bR,11cS)-10-hydroxy-14-(2,2,2-trifluoroacetyl)-1,2,3a,4,5,6,7,11c-octahydro-3H-6,11b-(epiminoethano)-1,5a-methanonaphtho[1,2-e]indole-3-carboxylic acid 2,2,2-trichloroethyl (1.46 g) obtained above was added and dissolved in acetic acid (25 mL). Zinc powder (1.31 g, 20 mmol) was added to the obtained solution, and the resulting mixture was stirred at room temperature for two hours. The reaction solution was filtered through celite, and an excess of zinc powder was distilled off. The filtrate was concentrated under reduced pressure and then azeotroped with toluene. The residue was diluted with a saturated sodium bicarbonate aqueous solution (30 mL), followed by extraction with chloroform (30 mL × 3). The collected organic layer was dried over anhydrous sodium sulfate. Thereafter, the insoluble matter was filtered off, and the filtrate was concentrated under reduced pressure. The obtained residue was subjected to column chromatography (amino silica gel, 16 g) using ethyl acetate and methanol (concentration gradient: 0% to 30%) as an elution solvent to obtain the title compound (215.7 mg, 3 step overall yield 27%) as a pale yellow foam-like substance.
¹ H NMR (CDCl₃, 400MHz) : δ = 6.96-7.06 (m, 1H), 6.64-6.72 (m, 1H), 6.52-6.58 (m, 1H), 5.90 (br s, 1H), 4.90 (d, 0.5H, J = 6.8Hz), 4.34 (dd, 0.5H, J = 13.8Hz, 6.5Hz), 4.18-4.24 (m, 0.5H), 2.72-3.81 (m, 8.5H), 2.21-2.45 (m, 1H), 1.46-2.00 (m, 3H), 0.99-1.43 (m, 4H). NH is not seen.

### Example 1

### Synthesis of (1S,3aR,5aS,6R,11bR,11cS)-3-(L-prolyl)-14-(cyclopropylmethyl)-10-hydroxy-2,3,3a,4,5,6,7,11c-octahydro-1H-6,11b-(epiminoethano)-1,5a-methanonaphtho[1,2-e]indole dihydrochloride

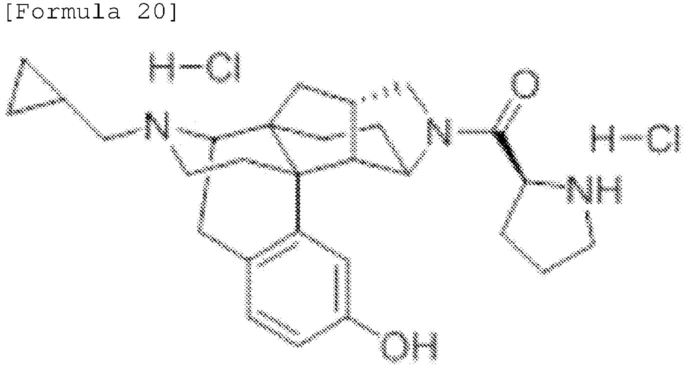

### (1) (S)-2-((1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole-3-carbonyl) pyrrolidine-1-tert-butyl carboxylate

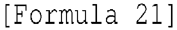

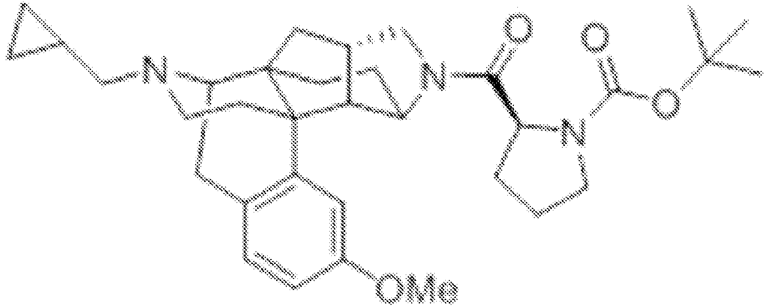

Into a 50 mL round bottom flask, (1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole (20 mg, 55 µmol) synthesized by a method in Example 67 of Patent Literature WO 2013/035833, (tert-butoxycarbonyl)-L-proline (14 mg, 0.66 mmol) obtained in Reference Example 2, and diisopropylethylamine (14.3 µL, 82 µmol) were added and suspended in dichloromethane (1 mL). Thereafter, HATU (25 mg, 0.66 mmol) was added thereto, and the resulting mixture was stirred at room temperature for 30 hours. A saturated sodium bicarbonate aqueous solution was added to the reaction solution, followed by extraction with chloroform three times. The collected organic layer was dried over sodium sulfate, and the insoluble matter was filtered off. Thereafter, the filtrate was concentrated under reduced pressure. The obtained residue was subjected to column chromatography (silica gel, 10 g) using methanol and chloroform (concentration gradient: 0% to 20%) as an elution solvent to obtain (S)-2-((1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole-3-carbonyl) pyrrolidine-1-tert-butyl carboxylate (28.5 mg, 92%).
¹H NMR (CDCl₃, 400MHz) : δ = 7.00-7.09 (m, 1H), 6.63-6.73 (m, 2H), 3.72-4.58 (m, 7H), 2.63-3.65 (m, 9H), 0.70-2.45 (m, 24H), 0.42-0.59 (m, 2H), 0.04-0.23 (m, 2H).

### (2) (1S,3aR,5aS,6R,11bR,11cS)-3-(L-prolyl)-14-(cyclopropylmethyl)-10-methoxy-2,3,3a,4,5,6,7,11c-octahydro-1H-6,11b-(epiminoethano)-1,5a-methanonaphtho[1,2-e]indole

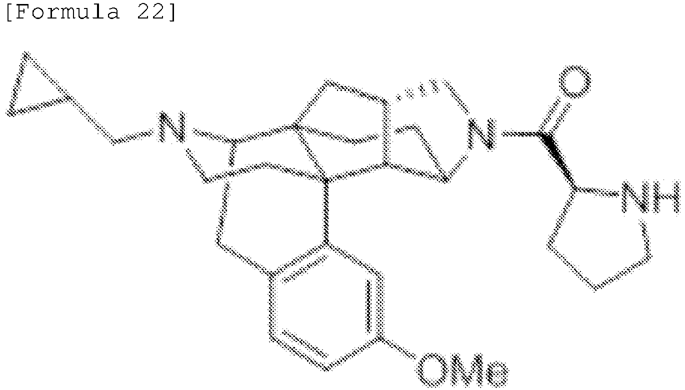

(S)-2-((1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole-3-carbonyl) pyrrolidine-1-tert-butyl carboxylate obtained above was dissolved in dichloromethane (1 mL), and trifluoroacetic acid (1 mL) was added thereto in an ice bath. The resulting mixture was stirred at room temperature for two hours. A potassium carbonate aqueous solution was added to the reaction solution, followed by extraction with chloroform four times. The collected organic layer was dried over sodium sulfate, and the insoluble matter was filtered off. Thereafter, the filtrate was concentrated under reduced pressure to obtain (1S,3aR,5aS,6R,11bR,11cS)-3-(L-prolyl)-14-(cyclopropylmethyl)-10-methoxy-2,3,3a,4,5,6,7,11c-octahydro-1H-6,11b-(epiminoethano)-1,5a-methanonaphtho[1,2-e]indole.
¹H NMR (CDCl₃, 400MHz) : δ = 7.06 (d, 0.3H, J = 8.2Hz), 7.02 (d, 0.7H, J = 8.2Hz), 6.64-6.72 (m, 2H), 2.70-4.54 (m, 15H), 0.75-2.59 (m, 17H), 0.43-0.51 (m, 2H), 0.06-0.12 (m, 2H).

### (3) (1S,3aR,5aS,6R,11bR,11cS)-3-(L-prolyl)-14-(cyclopropylmethyl)-10-hydroxy-2,3,3a,4,5,6,7,11c-octahydro-1H-6,11b-(epiminoethano)-1,5a-methanonaphtho[1,2-e]indole

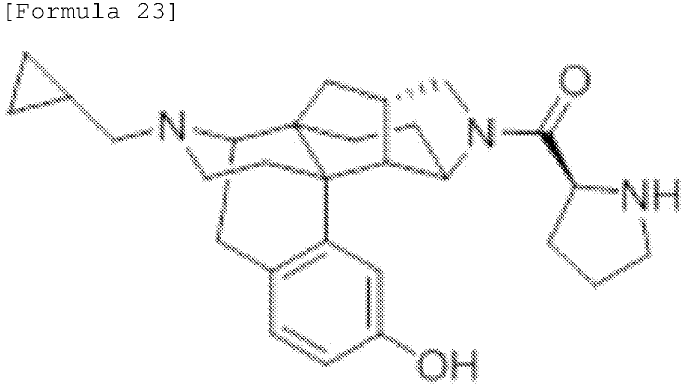

(1S,3aR,5aS,6R,11bR,11cS)-3-(L-prolyl)-14-(cyclopropylmethyl)-10-methoxy-2,3,3a,4,5,6,7,11c-octahydro-1H-6,11b-(epiminoethano)-1,5a-methanonaphtho[1,2-e]indole obtained above was dissolved in dichloromethane (1 mL). A 1.0 M boron tribromide/dichloromethane solution (0.25 mL, 0.25 mmol) was added thereto under ice cooling, and the resulting mixture was stirred for one hour. To the reaction solution, a 1.4 N ammonia/methanol solution was added under ice cooling, and the reaction was stopped. Thereafter, the resulting solution was concentrated under reduced pressure. The obtained residue was subjected to preparative TLC using methanol containing 10% concentrated ammonia water and chloroform (concentration: 10%) as a developing solvent to obtain (1S,3aR,5aS,6R,11bR,11cS)-3-(L-prolyl)-14-(cyclopropylmethyl)-10-hydroxy-2,3,3a,4,5,6,7,11c-octahydro-1H-6,11b-(epiminoethano)-1,5a-methanonaphtho[1,2-e]indole.
¹H NMR (CDCl₃, 400MHz) : δ = 6.95 (d, 0.6H, J = 8.2Hz), 6.81 (d, 0.4H, J = 8.2Hz), 6.62 (dd, 0.6H, J = 8.2Hz, 2.8Hz), 6.43-6.48 (m, 1.4H), 4.47-4.51 (m, 0.6H), 3.69-4.10 (m, 1.4H), 3.20-3.61 (m, 4H), 2.53-3.12 (m, 7H), 2.17-2.37 (m, 3H), 1.59-2.10 (m, 5.6H), 0.62-1.43 (m, 7.4H), 0.41-0.53 (m, 2H), 0.02-0.16 (m, 2H). OH is not seen.

### (4) (1S,3aR,5aS,6R,11bR,11cS)-3-(L-prolyl)-14-(cyclopropylmethyl)-10-hydroxy-2,3,3a,4,5,6,7,11c-octahydro-1H-6,11b-(epiminoethano)-1,5a-methanonaphtho[1,2-e]indole dihydrochloride

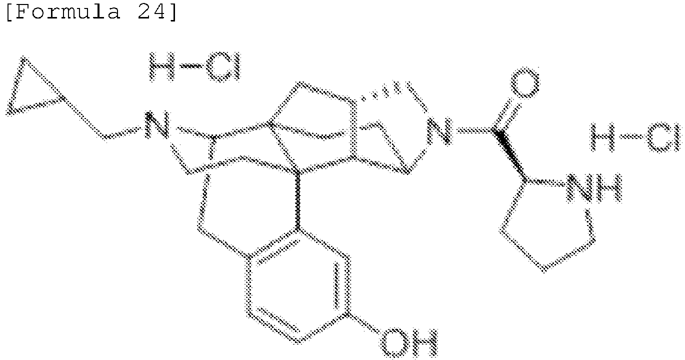

Into a 10 mL test tube, (1S,3aR,5aS,6R,11bR,11cS)-3-(L-prolyl)-14-(cyclopropylmethyl)-10-hydroxy-2,3,3a,4,5,6,7,11c-octahydro-1H-6,11b-(epiminoethano)-1,5a-methanonaphtho[1,2-e]indole obtained above and ethyl acetate were added, followed by extraction with 1 N hydrochloric acid. The aqueous layer was concentrated under reduced pressure. The obtained residue was dried under reduced pressure to obtain the title compound (12 mg, 3 steps 44%) as pale yellow amorphous.
¹H NMR (CD₃OD, 400MHz) : δ = 7.06-7.81 (m, 1H), 6.66-6.81 (m, 2H), 2.90-5.59 (m, 14H), 2.71-2.88 (m, 1H), 2.47-2.65 (m, 1H), 1.80-2.29 (m, 5H), 1.47-1.75 (m, 3H), 1.06-1.43 (m, 3H), 0.66-0.97 (m, 3H), 0.43-0.62 (m, 2H). NH, OH, and 2HCl are not seen.
MS (ESI+) : 448.23.

### Example 2

### Synthesis of (1S,3aR,5aS,6R,11bR,11cS)-3-(D-prolyl)-14-(cyclopropylmethyl)-10-hydroxy-2,3,3a,4,5,6,7,11c-octahydro-1H-6,11b-(epiminoethano)-1,5a-methanonaphtho[1,2-e]indole dihydrochloride

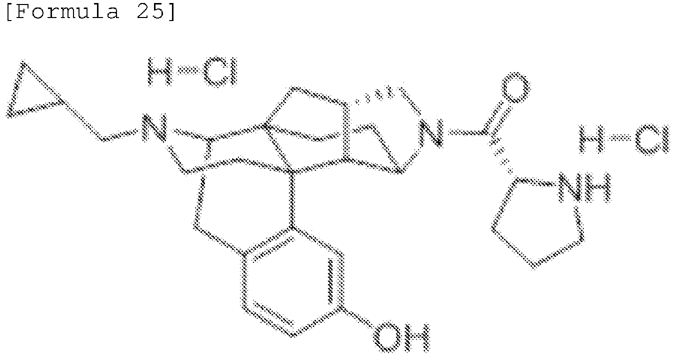

### (1) (R)-2-((1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole-3-carbonyl) pyrrolidine-1-tert-butyl carboxylate

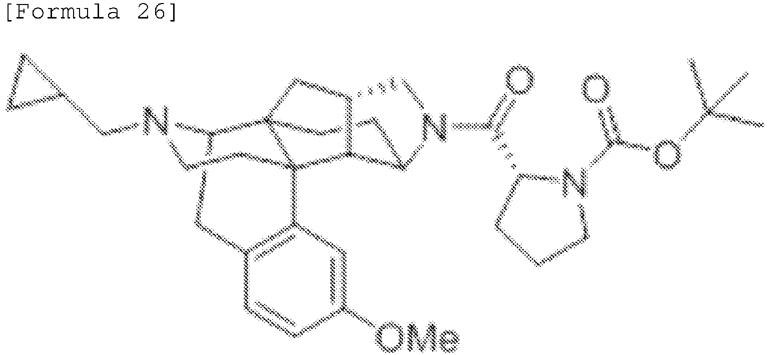

In a similar manner to Example 1(1), (1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole (20 mg, 55 µmol), (tert-butoxycarbonyl)-D-proline (14 mg, 0.66 mmol) obtained in Reference Example 3, and diisopropylethylamine (14.3 µL, 82 µmol) were added and suspended in dichloromethane (1 mL). Thereafter, HATU (25 mg, 0.66 mmol) was added thereto, and the resulting mixture was stirred at room temperature for one hour. A saturated sodium bicarbonate aqueous solution was added to the reaction solution, followed by extraction with chloroform three times. The collected organic layer was dried over sodium sulfate, and the insoluble matter was filtered off. Thereafter, the filtrate was concentrated under reduced pressure. The obtained residue was subjected to column chromatography (silica gel, 10 g) using methanol and chloroform (concentration gradient: 0% to 10%) as an elution solvent to obtain (R)-2-((1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole-3-carbonyl) pyrrolidine-1-tert-butyl carboxylate (18.1 mg, 59%).
¹H NMR (CDCl₃, 400MHz) : δ = 7.06 (d, 0.3H, J = 8.2Hz), 7.02 (d, 0.7H, J = 8.2Hz), 6.59-6.73 (m, 2H), 2.62-4.68 (m, 16H), 0.68-2.41 (m, 24H), 0.42-0.59 (m, 2H), 0.05-0.23 (m, 2H).
(2) (1S,3aR,5aS,6R,11bR,11cS)-3-(D-prolyl)-14-(cyclopropylmethyl)-10-hydroxy-2,3,3a,4,5,6,7,11c-octahydro-1H-6,11b-(epiminoethano)-1,5a-methanonaphtho[1,2-e]indole

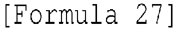

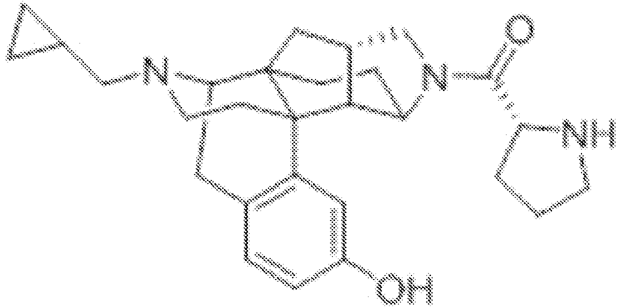

In a similar manner to Example 1(2), (R)-2-((1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole-3-carbonyl) pyrrolidine-1-tert-butyl carboxylate obtained above was dissolved in dichloromethane (1 mL), and trifluoroacetic acid (1 mL) was added thereto in an ice bath. The resulting mixture was stirred at room temperature for two hours. The reaction solution was concentrated, and then a potassium carbonate aqueous solution was added thereto, followed by extraction with chloroform four times. The collected organic layer was dried over sodium sulfate, and the insoluble matter was filtered off. Thereafter, the filtrate was concentrated under reduced pressure.

The obtained crude product was dissolved in dichloromethane (1 mL), and a 1.0 M boron tribromide/dichloromethane solution (0.16 mL, 0.16 mmol) was added thereto under ice cooling. The resulting mixture was stirred for one hour. To the reaction solution, a 1.4 N ammonia/methanol solution was added under ice cooling, and the reaction was stopped. Thereafter, the resulting solution was concentrated under reduced pressure, and a potassium carbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained residue was subjected to preparative TLC using methanol containing 10% concentrated ammonia water and chloroform (concentration: 10%) as a developing solvent to obtain (1S,3aR,5aS,6R,11bR,11cS)-3-(D-prolyl)-14-(cyclopropylmethyl)-10-hydroxy-2,3,3a,4,5,6,7,11c-octahydro-1H-6,11b-(epiminoethano)-1,5a-methanonaphtho[1,2-e]indole.
¹H NMR (CDCl₃, 400MHz) : δ = 6.90-6.96 (m, 1H), 6.57-6.62 (m, 2H), 3.61-4.53 (m, 4H), 2.80-3.46 (m, 8H), 2.51-2.59 (m, 1H), 2.28-2.36 (m, 2H), 1.61-2.18 (m, 7H), 0.72-1.48 (m, 7H), 0.42-0.55 (m, 2H), 0.05-0.16 (m, 2H). OH is not seen.

### (3) (1S,3aR,5aS,6R,11bR,11cS)-3-(D-prolyl)-14-(cyclopropylmethyl)-10-hydroxy-2,3,3a,4,5,6,7,11c-octahydro-1H-6,11b-(epiminoethano)-1,5a-methanonaphtho[1,2-e]indole dihydrochloride

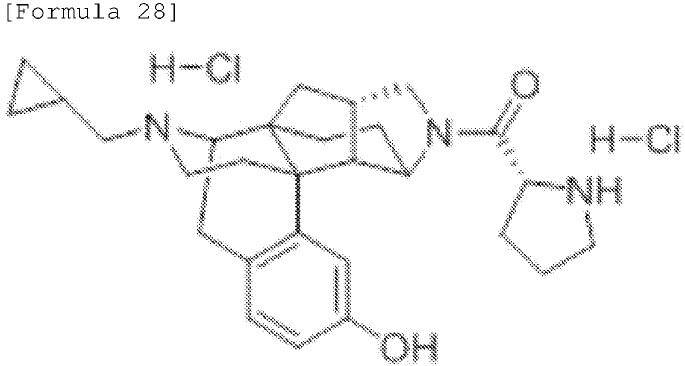

In a similar manner to Example 1(4), into a 10 mL test tube, (1S,3aR,5aS,6R,11bR,11cS)-3-(D-prolyl)-14-(cyclopropylmethyl)-10-hydroxy-2,3,3a,4,5,6,7,11c-octahydro-1H-6,11b-(epiminoethano)-1,5a-methanonaphtho[1,2-e]indole obtained above and ethyl acetate were added, followed by extraction with 1 N hydrochloric acid. The aqueous layer was concentrated under reduced pressure. The obtained residue was dried under reduced pressure to obtain the title compound (11 mg, 3 steps 68%) as pale yellow amorphous.
¹H NMR (CD₃OD, 400MHz) : δ = 7.09-7.14 (m, 1H), 6.68-6.80 (m, 2H), 2.99-5.24 (m, 15H), 2.72-2.86 (m, 1H), 1.81-2.63 (m, 6H), 1.09-1.74 (m, 5H), 0.68-0.94 (m, 3H), 0.45-0.58 (m, 2H). NH, OH, and 2HCl are not seen.
MS (ESI+) : 448.22.

### Example 3

### Synthesis of ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((S)-piperidin-2-yl) methanone dihydrochloride

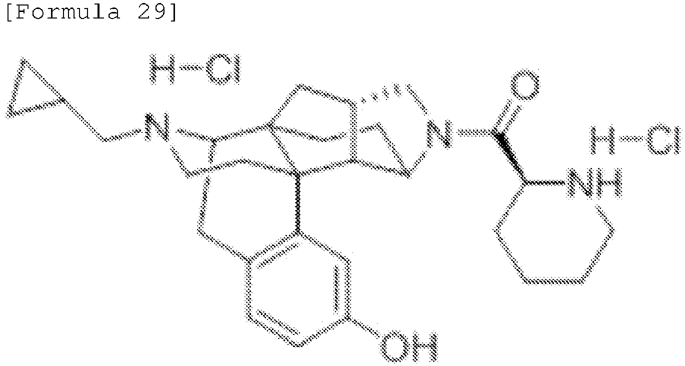

### (1) ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((S)-piperidin-2-yl) methanone

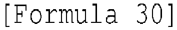

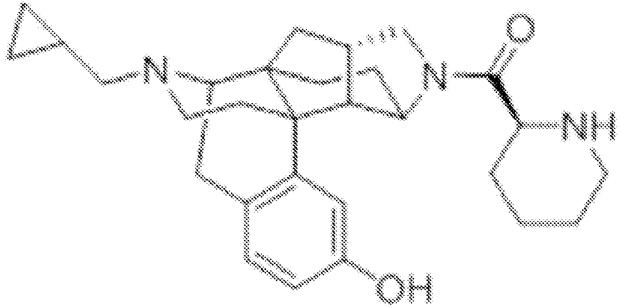

In a similar manner to Example 1(1), (1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole (20 mg, 55 µmol), (S)-1-(tert-butoxycarbonyl) piperidine-2-carboxylic acid (15 mg, 0.66 mmol) obtained in Reference Example 4, and diisopropylethylamine (14.3 µL, 82 µmol) were added and suspended in dichloromethane (1 mL). Thereafter, HATU (25 mg, 0.66 mmol) was added thereto, and the resulting mixture was stirred at room temperature for two hours. A saturated sodium bicarbonate aqueous solution was added to the reaction solution, followed by extraction with chloroform three times. The collected organic layer was dried over sodium sulfate, and the insoluble matter was filtered off. Thereafter, the filtrate was concentrated under reduced pressure. The obtained residue was subjected to column chromatography (silica gel, 10 g) using methanol and chloroform (concentration gradient: 0% to 10%) as an elution solvent to perform purification.

The compound obtained above was dissolved in dichloromethane (1 mL), and trifluoroacetic acid (1 mL) was added thereto in an ice bath. The resulting mixture was stirred at room temperature for 30 minutes. The reaction solution was concentrated, and then a potassium carbonate aqueous solution was added thereto, followed by extraction with chloroform three times. The collected organic layer was dried over sodium sulfate, and the insoluble matter was filtered off. Thereafter, the filtrate was concentrated under reduced pressure.

The obtained crude product was dissolved in dichloromethane (1 mL), and a 1.0 M boron tribromide/dichloromethane solution (0.12 mL, 0.12 mmol) was added thereto under ice cooling. The resulting mixture was stirred for one hour. To the reaction solution, a 1.4 N ammonia/methanol solution was added under ice cooling, and the reaction was stopped. Thereafter, the resulting solution was concentrated under reduced pressure, and a potassium carbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained residue was subjected to preparative TLC using methanol containing 10% concentrated ammonia water and chloroform (concentration: 10%) as a developing solvent to obtain ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((S)-piperidin-2-yl) methanone.
¹H NMR (CDCl₃, 400MHz) : δ = 6.95 (d, 0.3H, J = 8.7Hz), 6.92 (d, 0.7H, J = 8.7Hz), 6.51-6.61 (m, 2H), 2.82-4.50 (m, 11H), 2.55-2.71 (m, 2H), 2.26-2.39 (m, 2H), 0.73-2.07 (m, 16H), 0.42-0.52 (m, 2H), 0.04-0.18 (m, 2H). OH is not seen.

### (2) ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((S)-piperidin-2-yl) methanone dihydrochloride

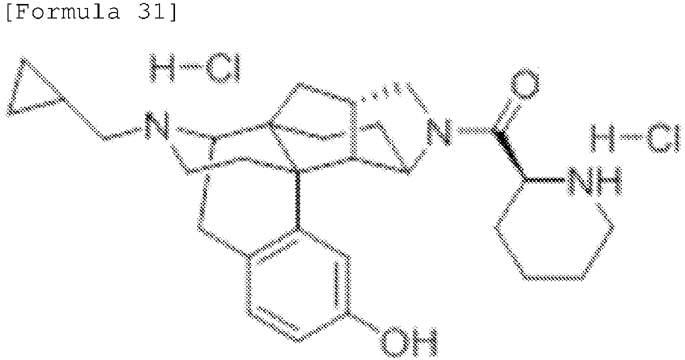

In a similar manner to Example 1(4), into a 10 mL test tube, ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl)((S)-piperidin-2-yl) methanone obtained above and ethyl acetate were added, followed by extraction with 1 N hydrochloric acid. The aqueous layer was concentrated under reduced pressure. The obtained residue was dried under reduced pressure to obtain the title compound (6.7 mg, 4 steps 51%) as pale yellow amorphous.
¹H NMR (CD₃OD, 400MHz) : δ = 7.07-7.16 (m, 1H), 6.78 (d, 0.3H, J = 2.3Hz), 6.68-6.75 (m, 1.7H), 2.88-5.11 (m, 15H), 2.72-2.85 (m, 1H), 2.08-2.31 (m, 2H), 1.49-2.05 (m, 9H), 0.68-1.40 (m, 5H), 0.44-0.59 (m, 2H). NH, OH, and 2HCl are not seen.
MS (ESI+) : 462.24.

### Example 4

### Synthesis of ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((R)-piperidin-2-yl) methanone dihydrochloride

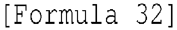

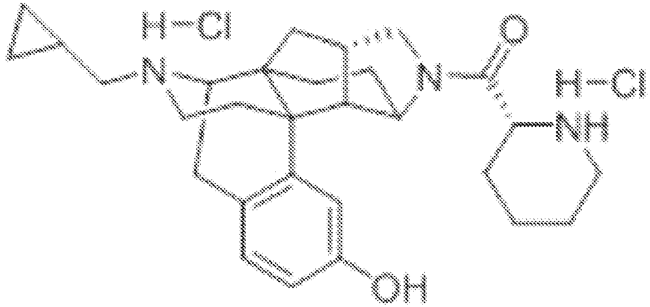

### (1) (R)-2-((1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole-3-carbonyl) piperidine-1-tert-butyl carboxylate

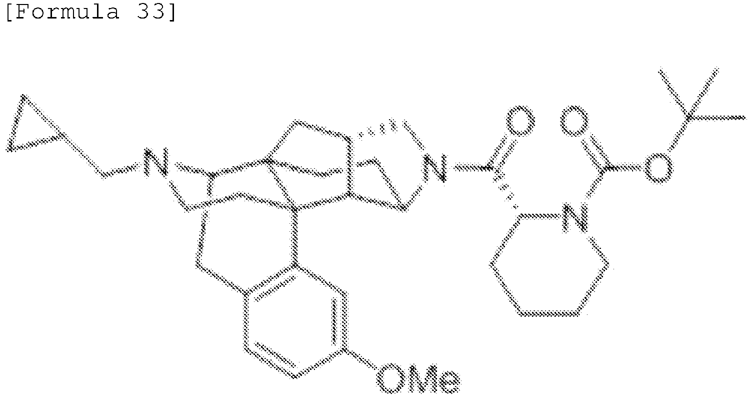

In a similar manner to Example 1(1), (1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole (30 mg, 82 µmol), (R)-1-(tert-butoxycarbonyl) piperidine-2-carboxylic acid (23 mg, 0.99 µmol) obtained in Reference Example 5, and diisopropylethylamine (21.5 µL, 0.12 mmol) were added and suspended in dichloromethane (1 mL). Thereafter, HATU (38 mg, 0.99 µmol) was added thereto, and the resulting mixture was stirred at room temperature for 23 hours. A saturated sodium bicarbonate aqueous solution was added to the reaction solution, followed by extraction with chloroform three times. The collected organic layer was dried over sodium sulfate, and the insoluble matter was filtered off. Thereafter, the filtrate was concentrated under reduced pressure. The obtained residue was subjected to column chromatography (silica gel, 10 g) using methanol and chloroform (concentration gradient: 0% to 15%) as an elution solvent to obtain (R)-2-((1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole-3-carbonyl) piperidine-1-tert-butyl carboxylate (46 mg, 98%).
¹H NMR (CDCl₃, 400MHz) : δ = 7.00-7.05 (m, 1H), 6.65-6.70 (m, 2H), 3.56-4.94 (m, 8H), 3.29-3.46 (m, 2H), 2.86-3.18 (m, 5H), 2.51-2.62 (m, 1H), 2.24-2.38 (m, 2H), 1.08-2.08 (m, 22H), 0.67-0.88 (m, 2H), 0.43-0.52 (m, 2H), 0.03-0.17 (m, 2H).

### (2) ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((R)-piperidin-2-yl) methanone

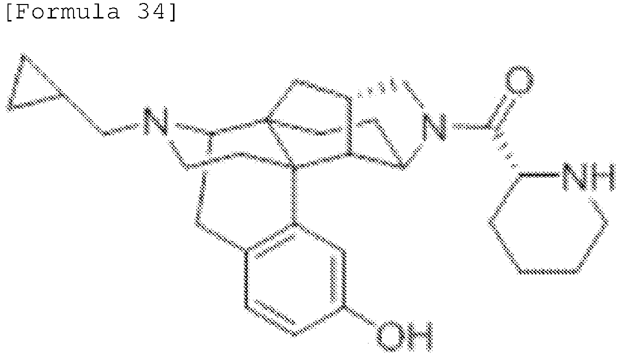

In a similar manner to Example 1(2), (R)-2-((1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole-3-carbonyl) piperidine-1-tert-butyl carboxylate obtained above was dissolved in dichloromethane (1 mL), and trifluoroacetic acid (1 mL) was added thereto in an ice bath. The resulting mixture was stirred at room temperature for 30 minutes. The reaction solution was concentrated, and then a potassium carbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained crude product was dissolved in dichloromethane (1 mL), and a 1.0 M boron tribromide/dichloromethane solution (0.40 mL, 0.40 mmol) was added thereto under ice cooling. The resulting mixture was stirred for one hour. To the reaction solution, a 1.4 N ammonia/methanol solution was added under ice cooling, and the reaction was stopped. Thereafter, the resulting solution was concentrated under reduced pressure, and a potassium carbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained residue was subjected to preparative TLC using methanol containing 10% concentrated ammonia water and chloroform (concentration: 10%) as a developing solvent to obtain ((1S,3aR,5aS,6R,11bR,11cS)-14-(cyclopropylmethy1)-10-hydroxy-1,2,3a,4,5,6,7,11c-octahydro-3H-6,11b-(epiminoethano)-1,5a-methanonaphtho[1,2-e]indol-3-yl) ((R)-piperidin-2-yl) methanone.
¹H NMR (CDCl₃, 400MHz) : δ = 6.92 (d, 0.6H, J = 8.2Hz), 6.87 (d, 0.4H, J = 8.2Hz), 6.74 (d, 0.6H, J = 2.3Hz), 6.63 (d, 0.4H, J = 2.3Hz), 6.57 (dd, 0.4H, J = 8.2Hz, 2.3Hz), 6.52 (dd, 0.6H, J = 8.2Hz, 2.3Hz), 4.52-4.64 (m, 1H), 3.43-3.79 (m, 3H), 2.63-3.36 (m, 8H), 2.44-2.60 (m, 1H), 2.21-2.40 (m, 2H), 0.64-2.12 (m, 16H), 0.38-0.54 (m, 2H), 0.03-0.16 (m, 2H). OH is not seen.

### (3) ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((R)-piperidin-2-yl) methanone dihydrochloride

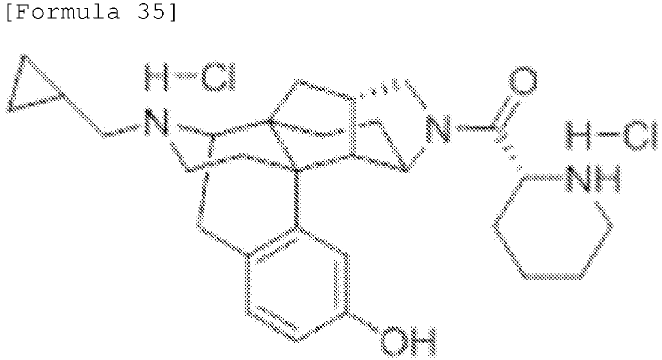

In a similar manner to Example 1(4), into a 10 mL test tube, ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((R)-piperidin-2-yl) methanone obtained above and ethyl acetate were added, followed by extraction with 1 N hydrochloric acid. The aqueous layer was concentrated under reduced pressure. The obtained residue was dried under reduced pressure to obtain the title compound (25 mg, 3 steps 59%) as pale yellow amorphous.
¹H NMR (CD₃OD, 400MHz) : δ = 7.12 (d, 0.2H, J = 8.2Hz), 7.11 (d, 0.8H, J = 8.7Hz), 6.66-6.82 (m, 2H), 2.91-5.16 (m, 15H), 2.72-2.85 (m, 1H), 2.09-2.30 (m, 1.8H), 1.46-2.03 (m, 10.2H), 1.09-1.34 (m, 1.2H), 0.70-0.94 (m, 2.8H), 0.45-0.59 (m, 2H). NH, OH, and 2HCl are not seen.
MS (ESI+) : 462.25.

### Example 5

### Synthesis of ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((R)-piperidin-3-yl) methanone dihydrochloride

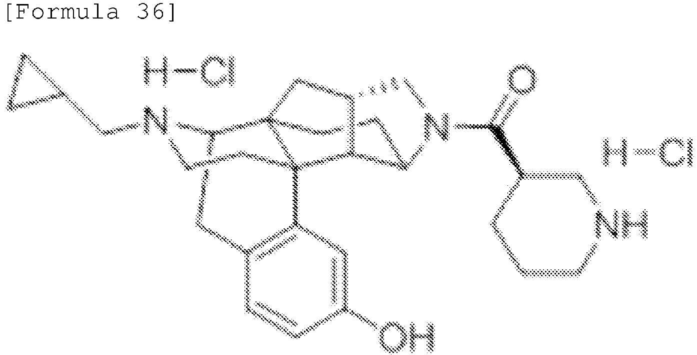

### (1) ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((R)-piperidin-3-yl) methanone

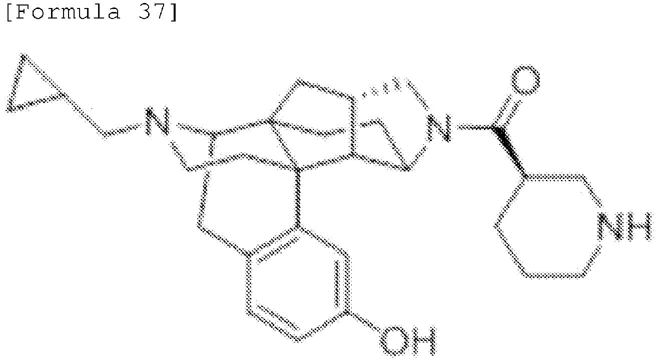

In a similar manner to Example 1(1), (1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole (20 mg, 55 µmol), (S)-1-(tert-butoxycarbonyl) piperidine-3-carboxylic acid (15 mg, 0.66 mmol) obtained in Reference Example 6, and diisopropylethylamine (14.3 µL, 82 µmol) were added and suspended in dichloromethane (1 mL). Thereafter, HATU (25 mg, 0.66 mmol) was added thereto, and the resulting mixture was stirred at room temperature for 16 hours. A saturated sodium bicarbonate aqueous solution was added to the reaction solution, followed by extraction with chloroform three times. The collected organic layer was dried over sodium sulfate, and the insoluble matter was filtered off. Thereafter, the filtrate was concentrated under reduced pressure. The obtained residue was subjected to column chromatography (silica gel, 10 g) using methanol and chloroform (concentration gradient: 0% to 10%) as an elution solvent to perform purification. The compound obtained above was dissolved in dichloromethane (1 mL), and trifluoroacetic acid (1 mL) was added thereto in an ice bath. The resulting mixture was stirred at room temperature for 20 minutes. The reaction solution was concentrated, and then a potassium carbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained crude product was dissolved in dichloromethane (1 mL), and a 1.0 M boron tribromide/dichloromethane solution (0.27 mL, 0.27 mmol) was added thereto under ice cooling. The resulting mixture was stirred for one hour. To the reaction solution, a 1.4 N ammonia/methanol solution was added under ice cooling, and the reaction was stopped. Thereafter, the resulting solution was concentrated under reduced pressure, and a potassium carbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained residue was subjected to preparative TLC using methanol containing 10% concentrated ammonia water and chloroform (concentration: 10%) as a developing solvent to obtain ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((R)-piperidin-3-yl) methanone.
¹HNMR (CDCl₃, 400MHz) : δ = 6.93 (d, 0.6H, J = 8.2Hz), 6.88 (d, 0.4H, J = 8.2Hz), 6.59-6.70 (m, 1.6H), 6.50 (dd, 0.4H, J = 8.2Hz, 2.2Hz), 3.84-4.52 (m, 2H), 2.64-3.52 (m, 10H), 2.54-2.59 (m, 1H), 2.24-2.36 (m, 2H), 0.72-2.10 (m, 16H), 0.40-0.55 (m, 2H), 0.04-0.17 (m, 2H). OH is not seen.

### (2) ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((R)-piperidin-3-yl) methanone dihydrochloride

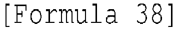

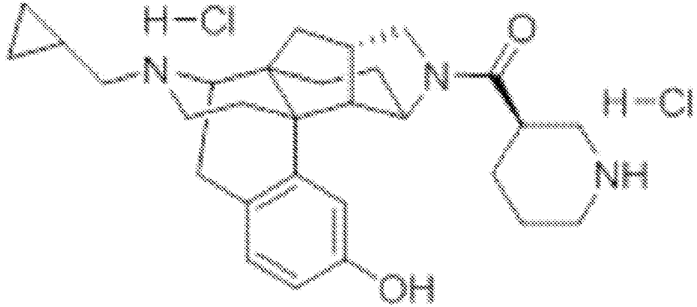

In a similar manner to Example 1(4), into a 10 mL test tube, ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((R)-piperidin-3-yl) methanone obtained above and ethyl acetate were added, followed by extraction with 1 N hydrochloric acid. The aqueous layer was concentrated under reduced pressure. The obtained residue was dried under reduced pressure to obtain the title compound (7 mg, 4 steps 23%) as pale yellow amorphous.
¹H NMR (CD₃OD, 400MHz) : δ = 7.12 (d, 0.3H, J = 8.2Hz), 7.11 (d, 0.7H, J = 9.2Hz), 6.68-6.78 (m, 2H), 2.70-5.08 (m, 18H), 2.09-2.23 (m, 1H), 1.47-2.08 (m, 8H), 1.08-1.44 (m, 2.3H), 0.69-0.94 (m, 2.7H), 0.44-0.59 (m, 2H). NH, OH, and 2HCl are not seen.
MS (ESI+) : 462.25.

### Example 6

### Synthesis of ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((S)-piperidin-3-yl) methanone dihydrochloride

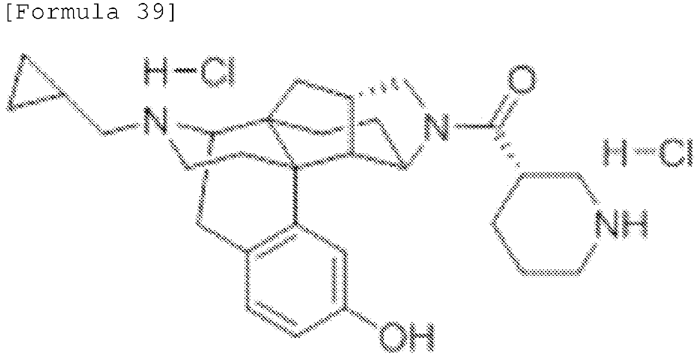

### (1) (S)-3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl) piperidine-1-tert-butyl carboxylate

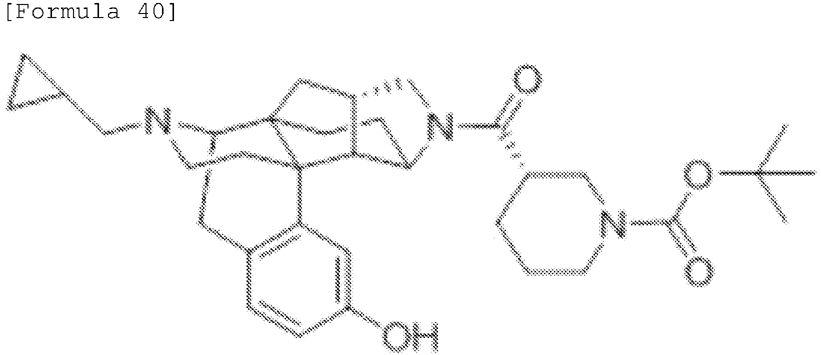

(1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indol-10-ol (20 mg, 56 µmol) synthesized in Reference Example 1, (S)-1-(tert-butoxycarbonyl) piperidine-3-carboxylic acid (101 mg, 0.44 mmol) obtained in Reference Example 7, and diisopropylethylamine (49 µL, 0.28 mmol) were added and suspended in dichloromethane (1 mL). Thereafter, HATU (47 mg, 0.12 mmol) was added thereto, and the resulting mixture was stirred at room temperature for one hour. To the reaction solution, a 1.4 N ammonia/methanol solution was added, and the reaction was stopped. Thereafter, the resulting solution was concentrated under reduced pressure, and a saturated sodium bicarbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained residue was subjected to preparative TLC using methanol and chloroform (concentration: 10%) as a developing solvent to obtain (S)-3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl) piperidine-1-tert-butyl carboxylate (27 mg, 86%).
¹H NMR (CDCl₃, 400MHz) : δ = 6.91-6.99 (m, 1.5H), 6.62-6.70 (m, 1.5H), 3.61-4.58 (m, 4H), 2.24-3.47 (m, 11H), 0.67-2.23 (m, 24H), 0.37-0.60 (m, 2H), 0.02-0.23 (m, 2H). OH is not seen.

### (2) ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((S)-piperidin-3-yl) methanone dihydrochloride

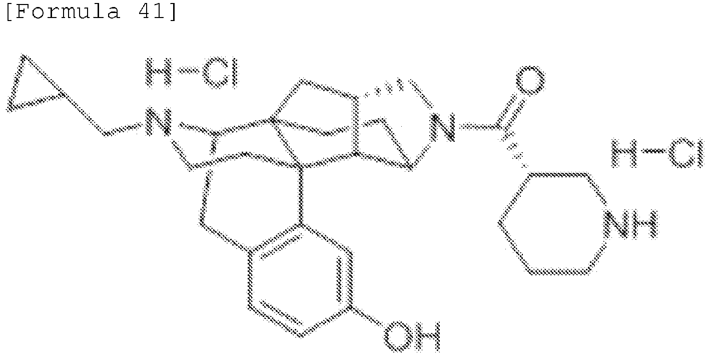

Into a 10 mL test tube, (S)-3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl) piperidine-1-tert-butyl carboxylate obtained above and ethyl acetate were added, followed by extraction with 1 N hydrochloric acid. The aqueous layer was concentrated under reduced pressure. The obtained residue was dried under reduced pressure to obtain the title compound (18 mg, 72%) as slightly brown amorphous.
¹H NMR (CD₃OD, 400MHz) : δ = 7.12 (d, 0.3H, J = 8.2Hz), 7.11 (d, 0.7H, J = 9.2Hz), 6.67-6.82 (m, 2H), 2.90-5.34 (m, 17H), 2.72-2.85 (m, 1H), 2.09-2.24 (m, 1H), 1.37-2.07 (m, 9H), 1.09-1.27 (m, 1.3H), 0.69-0.93 (m, 2.7H), 0.43-0.62 (m, 2H). NH, OH, and 2HCl are not seen.
MS (ESI+) : 462.26.

### Example 7

### Synthesis of ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) (piperidin-4-yl) methanone dihydrochloride

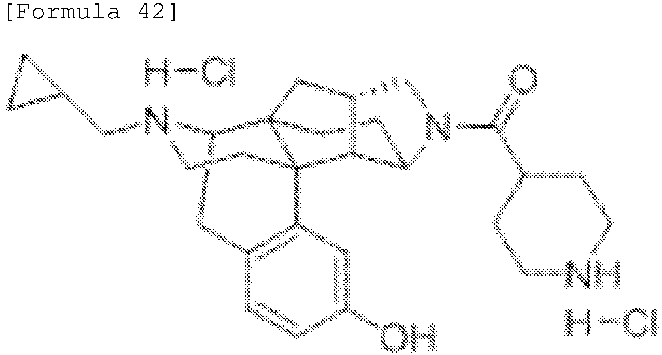

### (1) 4-((1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole-3-carbonyl) piperidine-1-tert-butyl carboxylate

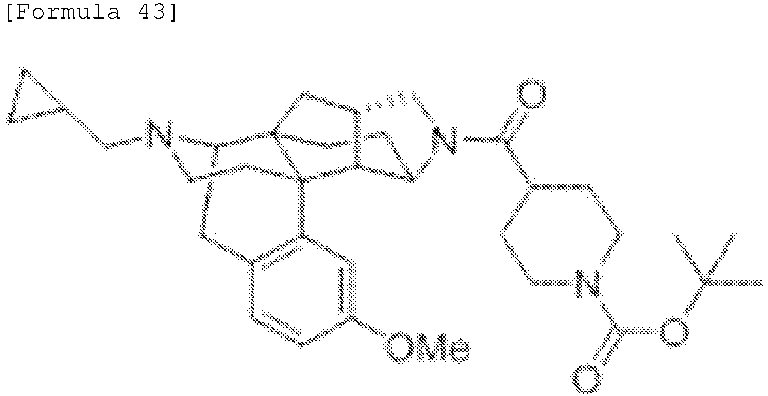

In a similar manner to Example 1(1), (1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole (20 mg, 55 µmol), 1-(tert-butoxycarbonyl) piperidine-4-carboxylic acid (15 mg, 66 µmol) obtained in Reference Example 8, and diisopropylethylamine (14.3 µL, 82 µmol) were added and suspended in dichloromethane (1 mL). Thereafter, HATU (25 mg, 66 µmol) was added thereto, and the resulting mixture was stirred at room temperature for one hour. A saturated sodium bicarbonate aqueous solution was added to the reaction solution, followed by extraction with chloroform three times. The collected organic layer was dried over sodium sulfate, and the insoluble matter was filtered off. Thereafter, the filtrate was concentrated under reduced pressure. The obtained residue was subjected to column chromatography (silica gel, 10 g) using methanol and chloroform (concentration gradient: 0% to 10%) as an elution solvent to obtain 4-((1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole-3-carbonyl) piperidine-1-tert-butyl carboxylate.
¹H NMR (CDCl₃, 400MHz) : δ = 7.01-7.09 (m, 1H), 6.64-6.73 (m, 2H), 3.76-4.53 (m, 7H), 3.33-3.60 (m, 11H), 0.72-2.08 (m, 24H), 0.42-0.62 (m, 2H), 0.05-0.27 (m, 2H).

### (2) ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) (piperidin-4-yl) methanone

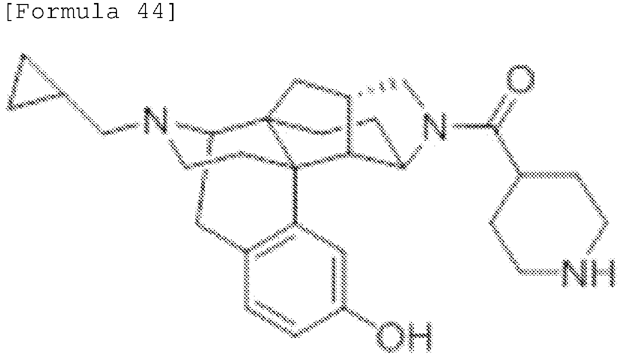

In a similar manner to Example 1(2), 4-((1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole-3-carbonyl) piperidine-1-tert-butyl carboxylate obtained above was dissolved in dichloromethane (1 mL), and trifluoroacetic acid (1 mL) was added thereto in an ice bath. The resulting mixture was stirred at room temperature. The reaction solution was concentrated, and then a potassium carbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained crude product was dissolved in dichloromethane (1 mL), and a 1.0 M boron tribromide/dichloromethane solution (0.28 mL, 0.28 mmol) was added thereto under ice cooling. The resulting mixture was stirred for one hour. To the reaction solution, a 1.4 N ammonia/methanol solution was added under ice cooling, and the reaction was stopped. Thereafter, the resulting solution was concentrated under reduced pressure, and a potassium carbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained residue was subjected to preparative TLC using methanol containing 10% concentrated ammonia water and chloroform (concentration: 10%) as a developing solvent to obtain ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) (piperidin-4-yl) methanone.
¹H NMR (CDCl₃, 400MHz) : δ = 6.89-6.94 (m, 1H), 6.56-6.63 (m, 2H), 3.84-4.48 (m, 2H), 2.49-3.57 (m, 11H), 2.21-2.34 (m, 2H), 0.72-2.08 (m, 16H), 0.37-0.52 (m, 2H), 0.02-0.13 (m, 2H). OH is not seen.

### (3) ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) (piperidin-4-yl) methanone dihydrochloride

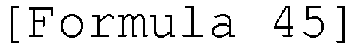

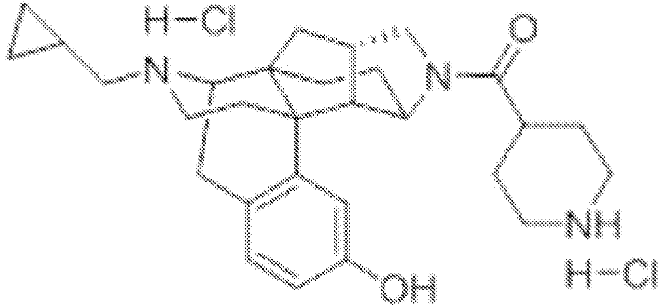

In a similar manner to Example 1(4), into a 10 mL test tube, ((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl)(piperidin-4-yl) methanone obtained above and ethyl acetate were added, followed by extraction with 1 N hydrochloric acid. The aqueous layer was concentrated under reduced pressure. The obtained residue was dried under reduced pressure to obtain the title compound (9 mg, 4 steps 32%) as pale yellow amorphous.
¹H NMR (CD₃OD, 400MHz) : δ = 7.07-7.15 (m, 1H), 6.78 (d, 0.3H, J = 2.3Hz), 6.67-6.76 (m, 1.7H), 2.68-5.24 (m, 18H), 2.09-2.24 (m, 1H), 1.46-2.07 (m, 8H), 0.67-1.43 (m, 5H), 0.44-0.60 (m, 2H). NH, OH, and 2HCl are not seen.
MS (ESI+) : 462.24.

### Example 8

### Synthesis of 4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indol-3-carbonyl) piperidine-2,6-dione

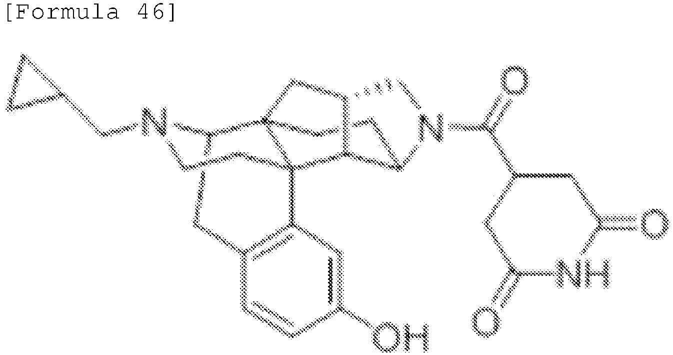

In a similar manner to Example 6(1), (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indol-10-ol (34 mg, 97 µmol) synthesized in Reference Example 1, 2,6-dioxopiperidine-4-carboxylic acid (34 mg, 0.22 mmol), and triethylamine (70 µL, 0.50 mmol) were added and suspended in THF (1.5 mL). Thereafter, HATU (125 mg, 0.33 mmol) and DMA (100 µL) were added thereto, and the resulting mixture was stirred at room temperature for two days. To the reaction solution, a 2 N ammonia/methanol solution (1 mL) was added, and the reaction was stopped. Thereafter, the reaction solution was diluted with 6% ammonia water (20 mL), followed by extraction with ethyl acetate three times. The collected organic layer was dried over sodium sulfate, and the insoluble matter was filtered off. Thereafter, the filtrate was concentrated under reduced pressure. The obtained residue was subjected to column chromatography (amino silica gel) using methanol and chloroform (concentration gradient: 0% to 50%) as an elution solvent. The obtained fraction was concentrated and then dissolved in methanol (0.2 mL). Thereafter, tert-butyl methyl ether (10 mL) was added thereto to obtain a powder. The obtained powder was filtered and then dried at 80°C for 16 hours to obtain the title compound (33 mg, 70%).
¹H NMR (CD₃OD, 400MHz) : δ = 6.94-6.98 (m, 1H), 6.55-6.66 (m, 2H), 4.33-4.42 (m, 1H), 3.86-3.97 (m, 1H), 3.69 (d, 0.6H, J = 12Hz), 3.26-3.49 (m, 4.4H), 3.12-3.21 (m, 2H), 2.90-3.01 (m, 2.5H), 2.55-2.77 (m, 4.5H), 2.32-2.34 (m, 2H), 1.91-2.10 (m, 2H), 1.69-1.83 (m, 1H), 1.38-1.55 (m, 1H), 1.13-1.30 (m, 2H), 0.78-0.86 (m, 2H), 0.43-0.53 (m, 2H), 0.07-0.17 (m, 2H). NH and OH are not seen.
MS (ESI+) : 490.21.

### Example 9

### Synthesis of (1S,3aR,5aS,6R,11bR,11cS)-14-(cyclopropylmethyl)-10-hydroxy-3-(methyl-D-prolyl)-2,3,3a,4,5,6,7,11c-octahydro-1H-6,11b-(epiminoethano)-1,5a-methanonaphtho[1,2-e]indol

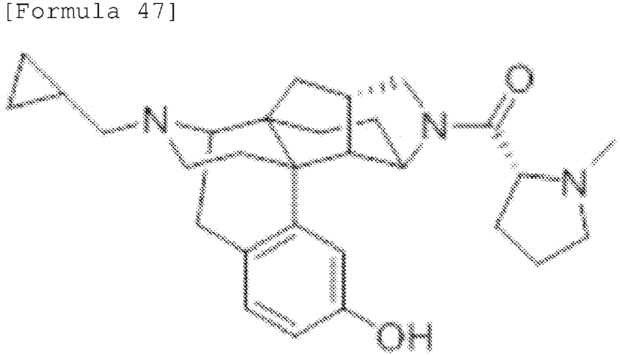

In a similar manner to Example 6(1), (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indol-10-ol (20 mg, 56 µmol) synthesized in Reference Example 1, methyl-D-proline (16 mg, 0.13 mmol) obtained in Reference Example 9, and diisopropylethylamine (50 µL, 0.29 mmol) were added and suspended in dichloromethane (1 mL). Thereafter, HATU (48 mg, 0.13 mmol) was added thereto, and the resulting mixture was stirred at room temperature for 14 hours. To the reaction solution, a 1.4 N ammonia/methanol solution was added, and the reaction was stopped. Thereafter, methanol was distilled off under reduced pressure, and a saturated sodium bicarbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained residue was subjected to preparative TLC using methanol containing 10% concentrated ammonia water and chloroform (concentration: 10%) as a developing solvent to obtain the title compound (13 mg, 48%) as pale yellow amorphous.
¹H NMR (CD₃OD, 400MHz) : δ = 6.97 (d, 0.3H, J = 8.2Hz), 6.97 (d, 0.7H, J = 8.2Hz), 6.53-6.67 (m, 2H), 2.84-5.19 (m, 10H), 2.54-2.66 (m, 1H), 1.68-2.46 (m, 13H), 1.05-1.54 (m, 5H), 0.77-0.96 (m, 2H), 0.43-0.57 (m, 2H), 0.07-0.20 (m, 2H). OH is not seen.
MS(ESI+) : 462.26.

### Example 10

### Synthesis of (R)-5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indol-3-carbonyl) pyrrolidin-2-one

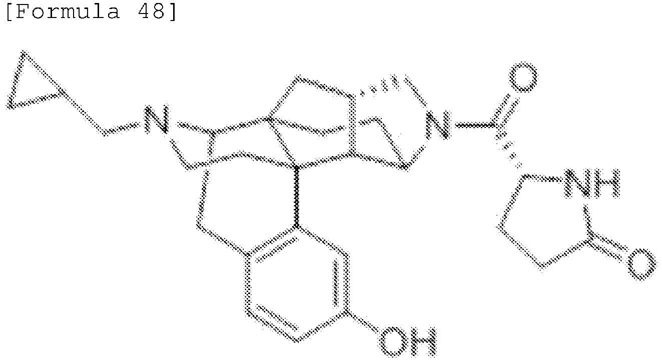

In a similar manner to Example 6(1), (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indol-10-ol (20 mg, 56 µmol) synthesized in Reference Example 1, (R)-5-oxopyrrolidine-2-carboxylic acid (16 mg, 0.13 mmol), and diisopropylethylamine (50 µL, 0.29 mmol) were added and suspended in dichloromethane (1 mL). Thereafter, HATU (48 mg, 0.13 mmol) was added thereto, and the resulting mixture was stirred at room temperature for 14 hours. To the reaction solution, a 1.4 N ammonia/methanol solution was added, and the reaction was stopped. Thereafter, methanol was distilled off under reduced pressure, and a saturated sodium bicarbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained residue was subjected to preparative TLC using methanol and chloroform (concentration: 10%) as a developing solvent to obtain the title compound (15 mg, 58%) as pale yellow amorphous.
¹H NMR (CD₃OD, 400MHz) : δ = 6.97 (d, 0.4H, J = 8.2Hz), 6.96 (d, 0.6H, J = 8.2Hz), 6.65 (d, 0.4H, J = 2.3Hz), 6.62 (d, 0.6H, J = 2.3Hz), 6.55-6.60 (m, 1H), 2.87-5.06 (m, 9H), 1.67-2.68 (m, 10H), 1.02-1.58 (m, 5H), 0.74-0.96 (m, 2H), 0.43-0.58 (m, 2H), 0.06-0.21 (m, 2H). NH and OH are not seen.
MS (ESI+) : 462.22.

### Example 11

### Synthesis of (R)-5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indol-3-carbonyl)-1-methylpyrrolidin-2-one

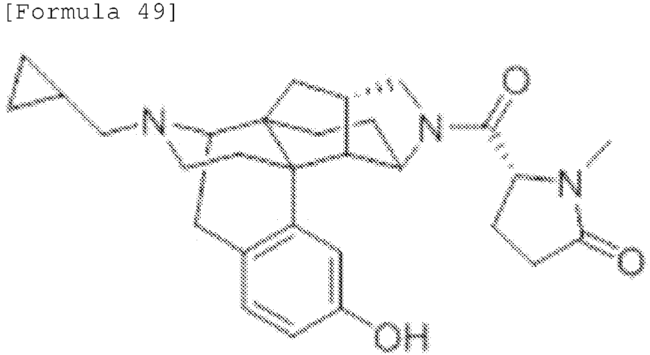

In a similar manner to Example 6(1), (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indol-10-ol (15 mg, 43 µmol) synthesized in Reference Example 1, (R)-1-methyl-5-oxopyrrolidine-2-carboxylic acid obtained in Reference Example 10, and diisopropylethylamine (37 µL, 0.21 mmol) were added and suspended in dichloromethane (1 mL) and DMF (1 mL). Thereafter, HATU (36 mg, 94 µmol) was added thereto, and the resulting mixture was stirred at room temperature for three hours. To the reaction solution, a 1.4 N ammonia/methanol solution was added, and the reaction was stopped. Thereafter, methanol was distilled off under reduced pressure, and a saturated sodium bicarbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained residue was subjected to preparative TLC using methanol and chloroform (concentration: 10%) as a developing solvent to obtain the title compound (4 mg, 22%) as pale yellow amorphous.
¹H NMR (CD₃OD, 400MHz) : δ = 6.98 (d, 0.3H, J = 8.2Hz), 6.97 (d, 0.7H, J = 8.2Hz), 6.67 (d, 0.3H, J = 2.8Hz), 6.62 (d, 0.7H, J = 2.8Hz), 6.56-6.60 (m, 1H), 2.85-5.16 (m, 10H), 2.74 (s, 2.1H), 2.73 (s, 0.9H), 1.69-2.67 (m, 9H), 0.78-1.65 (m, 7H), 0.43-0.58 (m, 2H), 0.07-0.23 (m, 2H). OH is not seen.
MS(ESI+) : 476.24.

### Example 12

### Synthesis of ((1S,3aR,5aS,6R,11bR,11cS)-14-(cyclopropylmethyl)-10-hydroxy-1,2,3a,4,5,6,7,11c-octahydro-3H-6,11b-(epiinoetano)-1,5a-methanonaphtho[1,2-e]indol-3-yl) ((R)-tetrahydrofuran-2-yl) methanone

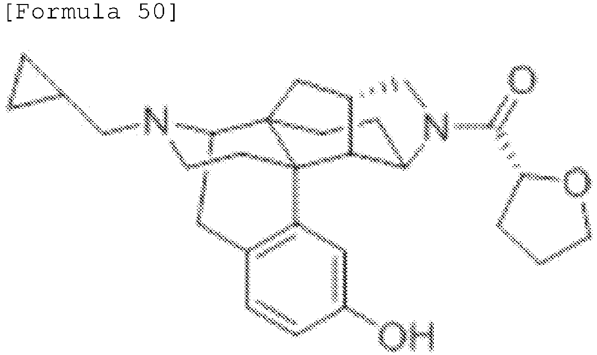

In a similar manner to Example 6(1), (1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indol-10-ol (15 mg, 43 µmol) synthesized in Reference Example 1, (R)-tetrahydrofuran-2-carboxylic acid (9 µL, 94 µmol), and diisopropylethylamine (37 µL, 0.21 mmol) were added and suspended in dichloromethane (1 mL). Thereafter, HATU (36 mg, 94 µmol) was added thereto, and the resulting mixture was stirred at room temperature for three days. To the reaction solution, a 1.4 N ammonia/methanol solution was added under ice cooling, and the reaction was stopped. Thereafter, methanol was distilled off under reduced pressure, and a saturated sodium bicarbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained residue was subjected to preparative TLC using methanol and chloroform (concentration: 5%) as a developing solvent to obtain the title compound (12 mg, 61%) as slightly brown amorphous.
¹H NMR (CD₃OD, 400MHz) : δ = 6.973 (d, 0.3H, J = 8.2H), 6.965 (d, 0.7H, J = 8.2Hz), 6.65 (d, 0.3H, J = 2.3Hz), 6.63 (d, 0.7H, J = 2.3Hz), 6.55-6.61 (m, 1H), 2.85-5.08 (m, 11H), 2.51-2.68 (m, 1H), 2.31-2.46 (m, 2H), 1.67-2.29 (m, 7H), 1.05-1.56 (m, 5H), 0.73-0.94 (m, 2H), 0.42-0.59 (m, 2H), 0.07-0.25 (m, 2H). OH is not seen.
MS (ESI+) : 449.24.

### Example 13

### Synthesis of (R)-5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl) dihydrofuran-2(3H)-one

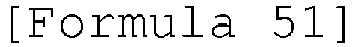

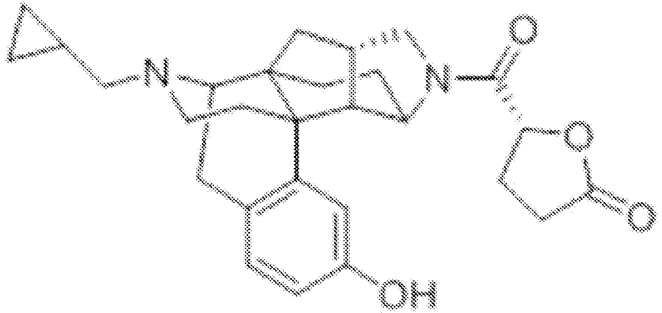

In a similar manner to Example 6(1), (1S,3aR,5aS,6R,11bR,11cS)-14-(cyclopropylmethyl)-2,3,3a,4,5,6, 7,11c-octahydro-1H-6,11b-(epiminoethano)-1,5a-methanonaphtho[1,2-e]indol-10-ol (20 mg, 57 µmol) synthesized in Reference Example 1, (R)-5-oxotetrahydrofuran-2-carboxylic acid (16 mg, 0.13 mmol), and diisopropylethylamine (50 µL, 0.29 mmol) were added and suspended in dichloromethane (1 mL) . Thereafter, HATU (48 mg, 0.13 mmol) was added thereto, and the resulting mixture was stirred at room temperature for two hours. To the reaction solution, a 1.4 N ammonia/methanol solution was added, and the reaction was stopped. Thereafter, methanol was distilled off under reduced pressure, and a saturated sodium bicarbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained residue was subjected to preparative TLC using methanol and chloroform (concentration: 10%) as a developing solvent to obtain the title compound (3 mg, 12%) as pale yellow amorphous.
¹H NMR (DMSO-d₆, 400MHz) : δ = 9.10 (s, 0.3H), 9.06 (s, 0.7H), 6.91 (d, 1H, J = 8.2Hz), 6.47-6.63 (m, 2H), 5.15-5.26 (m, 1H), 4.18-4.45 (m, 1H), 2.04-3.86 (m, 14H), 1.75-2.00 (m, 2H), 0.89-1.69 (m, 6H), 0.53-0.87 (m, 2H), 0.34-0.51 (m, 2H), -0.01-0.13 (m, 2H).
MS (ESI+) : 463.26.

### Example 14

### Synthesis of ((1S,3aR,5aS,6R,11bR,11cS)-10-hydroxy-1,2,3a,4,5,6,7,11c-octahydro-3H-6,11b-(epiinoetano)-1,5a-methanonaphtho[1,2-e]indol-3-yl) ((R)-tetrahydrofuran-2-yl) methanone

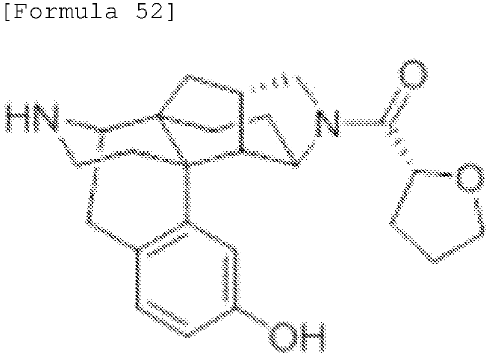

2,2,2-trifluoro-1-((1S,3aR,5aS,6R,11bR,11cS)-10-hydroxy-2,3,3a,4,5,6,7,11c-octahydro-1H-6,11b-(epiminoethano)-1,5a-methanonaphtho[1,2-e]indol-14-yl) ethan-1-one (39 mg, 0.10 mmol) synthesized in Reference Example 11(3), (R)-tetrahydrofuran-2-carboxylic acid (29 µL, 0.30 mmol), and triethylamine (84 µL, 0.60 mmol) were added and suspended in THF (1.5 mL) and DMA (0.1 mL). Thereafter, HATU (152 mg, 0.40 mmol) was added thereto, and the resulting mixture was stirred at room temperature for 14 hours. To the reaction solution, a 2 N ammonia/methanol solution was added, and the reaction was stopped. Thereafter, a saturated sodium bicarbonate aqueous solution was added thereto, followed by extraction with chloroform three times. The collected organic layer was dried over sodium sulfate, and the insoluble matter was filtered off. Thereafter, the filtrate was concentrated under reduced pressure. The obtained crude product was dissolved in methanol (2 mL). Thereafter, the resulting solution was added to sodium borohydride (76 mg, 2.0 mmol), and the resulting mixture was stirred at room temperature for one hour. To the reaction solution, 15 N ammonia water was added, and the reaction was stopped, followed by extraction with chloroform three times. The collected organic layer was dried over sodium sulfate, and the insoluble matter was filtered off. Thereafter, the filtrate was concentrated under reduced pressure. The obtained residue was subjected to preparative TLC using 15 N ammonia water, methanol, and chloroform (1 : 10 : 50) as a developing solvent to obtain the title compound (15 mg, 37%) as a white solid.
¹H NMR (CDCl₃, 400MHz) : δ = 6.92-7.00 (m, 1H), 6.55-6.66 (m, 2H), 4.43-4.59 (m, 2H), 2.61-4.10 (m, 10H), 0.75-2.28 (m, 14H). OH is not seen.
MS (ESI+) : 395.27.

### Example 15

### Opioid receptor function test

The functional activities of the compounds provided by the present invention on the µ, δ, and κ opioid receptors were investigated.

### Method:

The test was performed by using Lance Ultra cAMP Kit (PerkinElmer) according to a method predetermined for the kit. In the evaluation of the agonistic activity, CHO cells expressing each of the human opioid receptors (δ, µ, and κ, accession numbers and catalog numbers are mentioned below) and 10 µM of each test compound were reacted for 30 minutes in an assay buffer (1 x HBSS, 1 M HEPES, pH 7.4, 250 mM IBMX (isobutylmethylxanthine), 7.5% BSA) in the presence of forskolin. Subsequently, the cAMP detection reagent included in the kit was added, and 1 hour afterward, time-resolved fluorescence measurement was performed by using the EnVision plate reader (PerkinElmer). The test compounds and the control drugs (δ: SNC80, µ: DAMGO, κ: U-69593) were evaluated in a concentration range of 10-12 to 10-5 M, a dose-response curve of each test compound was obtained from the fluorescence values at 665 nm, and EC50 value and the Emax value were calculated. The Emax value was calculated as a ratio of the maximum reaction of the test compound to the maximum reaction of each control drug, which is taken as 100%.
SNC80:
   (+)-4-[(αR)-α-((2S,5R)-4-allyl-2,5-dimethyl-1-piperazinyl)-3-methoxybenzyl]-N,N-diethylbenzamide
DAMGO:
   [D-Ala²,N-MePhe⁴,Gly-ol]enkephalin U-69593:
(+)-(5α,7α,8β)-N-methyl-N-[7-(1-pyrrolidinyl)-1-oxaspiro[4.5]dec-8-yl]benzeneacetamide

### Accession number and catalog number

δ: Catalog No. CT 4607, accession No. NM-000911.2
µ: Catalog No. CT 4605, accession No. NM-000914
κ: Catalog No. CT 4606, accession No. NM-000912

### (ChanTest Corporation)

**[Table 1]**

| | δ receptor | | µ receptor | | κ receptor | |
|---|---|---|---|---|---|---|
| | EC_{bC} (nM) | Eₘₐₓ (%) | EC_{bC} (nM) | Eₘₐₓ (%) | EC_{bC} (nM) | Eₘₐₓ (%) |
| Example 2 | <3 | 95 | N.C. | 3.8* | N.C. | 3.8* |
| Example 4 | <3 | 82 | N.C. | 0.87* | N.C. | 5.0* |
| Example 6 | <3 | 74 | N.C. | 5.5* | <1 | 11 |
| Example 9 | <3 | 64 | N.C. | -0.6* | N.C. | 3.7* |
| Example 12 | <1 | 85 | >1 | 12 | N.C. | 8.7* |
| Example 13 | <3 | 70 | >1 | 35 | <1 | 13 |

| | | | | | | |
|---|---|---|---|---|---|---|
| N.C.: Since the maximum reaction was not reached at the highest concentration (10 µM), the ED₅₀ value was not calculated. *: Since the maximum reaction was not reached at the maximum concentration, a reaction rate at the highest concentration was indicated as a reference value. | | | | | | |

As shown in Table 1, it was confirmed that the compounds of the present invention have potent agonistic activities for the opioid δ receptor, but do not have agonistic activity or have only very weak agonistic activity for the µ and κ receptors.

### Example 16

### Mouse elevated plus maze test

### (Test method)

For the test, 5 to 6 weeks old C57BL/6N male mice were used. On a plus maze apparatus consisting of a wall-less running route (open arm, width 6 cm, length 30 cm) and a running route with a wall (closed arm, width 6 cm, length 30 cm, height of wall 15 cm), and having a height of 40 cm, a mouse was put so as to be directed to the running route with a wall, and allowed to spontaneously enter into the plus maze. Each test substance was dissolved in saline or 0.005 N HCl in saline, and subcutaneously administered on the back 30 minutes before the start of the test. At the time of the start of the test, video recording with a video camera was started, the time at which the mouse entered into the plus maze is considered to be the start of the test, and exploratory behavior was recorded for 5 minutes. On the basis of the video image, staying time in the running routes were determined, and wall-less running route staying time ratio (%) was calculated.

### (Test results)

As shown in Fig. 1, in this experiment, it has been confirmed that the compound described in Example 12 significantly increases the wall-less traveling path residence time rate with 0.3 mg/kg subcutaneous administration, and exhibits an anxiolytic effect.

### Example 17

### Rat elevated plus maze test

An anxiolytic effect of a compound provided by the present invention was examined using the rat elevated plus maze test.

### (Test method)

For the test, 7 to 9 weeks old Wistar male rats were used. On a plus maze apparatus consisting of a wall-less running route (width 10 cm, length 50 cm) and a running route with a wall (width 10 cm, length 50 cm, height of wall 30 cm), and having a height of 50 cm, a rat was put so as to be directed to the running route with a wall, and allowed to spontaneously enter into the plus maze, and exploratory behavior was observed for 5 minutes. Each test substance was dissolved in a 4.5% aqueous solution of cyclodextrin, and orally administered 2 hours before the start of the test. The test data were automatically analyzed by using video image action analysis software (Smart3.0, PanLab S.L., PanLab), and wall-less running route staying time ratio (%) was calculated.

### (Test results)

As shown in Fig. 2, in this experiment, it has been confirmed that the compound described in Example 12 significantly increases the wall-less traveling path residence time rate with 0.3 mg/kg oral administration, and exhibits an anxiolytic effect.

### Example 18

### Human ether-a-go-go related gene (hERG) potassium channel inhibition test

### (Test method)

The test was performed with Port-a-Patch automatic patch clump apparatus (Nanion Technologies) using hERG channel-stably expressing CHO cells (purchased from Channelopathy Foundation). The membrane potential of the cells was maintained at -80 mV, then there were applied a depolarization pulse at +20 mV for 1.5 seconds, and a following test pulse at -50 mV for 1.5 seconds at a frequency of 1 time per 10 seconds, and the hERG current was confirmed as a tail current induced by the test pulse. The test compound was dissolved in an extracellular fluid (137 mM NaCl, 4 mM KCl, 1.8 mM CaCl2, 1 mM MgCl2, 10 mM D(+)-glucose, 10 mM HEPES, pH 7.4), and the solution was refluxed at room temperature for 5 minutes. The inhibition ratio was obtained from the ratio of the tail current value observed after the compound was applied based on the tail current value observed before the compound was applied, which was taken as 100%. For the test, we used cells that showed a peak tail current value not smaller than 300 pA, tail current run-down smaller than 10% of the initial current value, and leak current smaller than 200 pA.

### (Test results)

The test results are shown in Table 7. As clearly seen from the results shown in Table 2, compounds described in Examples 2, 4, and 14 exhibited only a weak inhibitory effect.

**[Table 2]**

| Test compound | Concentration | hERG channel inhibitory effect |
|---|---|---|
| Example 2 | 10 µM | <50% |
| Example 4 | 10 µM | <50% |
| Example 12 | 1 µM | <50% |
| Example 13 | 1 µM | <50% |
| Example 14 | 10 µM | <50% |

### Example 19

Intracerebral migration test

### (Test method)

A Wistar male 5-9 week old rat was used for the test. A test substance was dissolved in saline or a 4.5% cyclodextrin aqueous solution, and 1 mol/L hydrochloric acid (0.5 to 2.0 v/v%) was added thereto as necessary. The test substance was subcutaneously administered to the rat at a dose of 3 or 10 mg/kg. Thirty minutes later, cerebrospinal fluid was collected from the cisterna magna under isoflurane anesthesia. Subsequently, laparotomy was performed, and blood was collected from the vena cava. Thereafter, the blood was centrifuged at 1500 × g at 4°C for 15 minutes to obtain plasma. The animal was euthanized promptly by aortic cutting. The concentrations of the test substance in cerebrospinal fluid and plasma were measured using LC-MS/MS.

### (Test results)

**[Table 3]**

| Test compound | Dosage (mg/kg, s.c.) | Concentration in cerebrospinal fluid (nmol/L) | Concentration in plasma (nmol/L) |
|---|---|---|---|
| Comparative compound | 10 | 3.6 | 2400 |
| Example 12 | 3 | 42 | 580 |

A comparative compound was a compound described in Example 12 of WO 2014/136305 (Patent Literature 6). Incidentally, the comparative compound had a minimum effective medicinal dose of 10 mg/kg, s.c. in the same test as the rat elevated plus maze test described in Example 17 here. The concentration in the cerebrospinal fluid at this time was almost equal to a Ki value of 1.2 nmol/L of binding affinity to the opioid δ receptor (measured by [³H]DPDPE competitive binding test in a mouse cerebral membrane fraction). Meanwhile, a compound described in Example 12 of the present application had a minimum effective medicinal dose of 0.3 mg/kg, p. o. Therefore, it has been revealed that the compound described in Example 12 exhibits a medicinal effect at a lower dose due to having excellent intracerebral migration.

### Description of Notations

In Figs. 1 and 2, the vertical axis indicates a ratio of residence time on a wall-less traveling path, and the horizontal axis indicates a test drug and a dosage thereof.

## Claims

1. A compound represented by the following general formula (I), 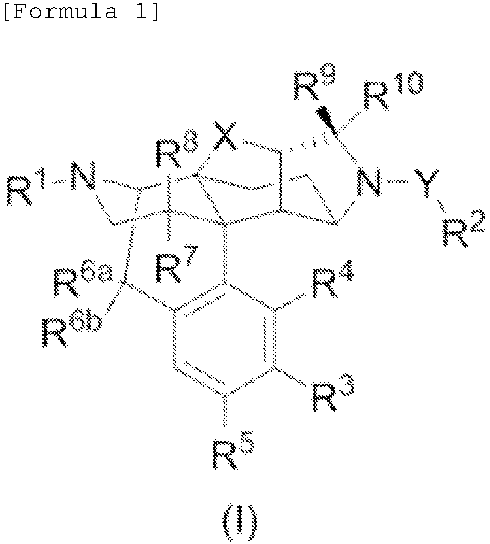
(wherein R¹ represents hydrogen; C₁₋₁₀ alkyl; C₆₋₁₀ aryl; C₂₋₆ alkenyl; cycloalkylalkyl where the cycloalkyl moiety has 3 to 6 carbon atoms, and the alkylene moiety has 1 to 5 carbon atoms; aralkyl where the aryl moiety has 6 to 10 carbon atoms and the alkylene moiety has 1 to 5 carbon atoms; C₃₋₆ cycloalkyl; or heteroarylalkyl where the heteroaryl moiety contains 1 to 4 heteroatoms selected from N, O, and S as ring-constituting atoms and the alkylene moiety has 1 to 5 carbon atoms,
R² represents a 4- to 7-membered saturated heterocycle containing one or two heteroatoms which may be the same or different and are selected from N, O, and S, and two or more carbon atoms as ring-constituting atoms,
R² binds to Y via a carbon atom which is a ring-constituting atom of R²,
R³, R⁴, and R⁵, which are the same or different, represent hydrogen; hydroxy; halogen; cyano; carbamoyl; C₁₋₆ alkoxy; C₆₋₁₀ aryloxy; C₁₋₆ alkanoyloxy; nitro; amino; C₁₋₈ alkylamino; C₆₋₁₀ arylamino; or acylamino where the aryl moiety has 2 to 6 carbon atoms,
R^{6a} and R^{6b}, which are the same or different, represent hydrogen; a fluorine atom, or hydroxy, or R^{6a} and R^{6b} combine together to represent = O,
R⁷ and R⁸, which are the same or different, represent hydrogen; fluorine; or hydroxy,
R⁹ and R¹⁰, which are the same or different, represent hydrogen; C₁₋₆ alkyl; C₆₋₁₀ aryl; heteroaryl containing 1 to 4 heteroatoms selected from N, O, and S as ring-constituting atoms; aralkyl where the aryl moiety has 6 to 10 carbon atoms and the alkylene moiety has 1 to 5 carbon atoms; heteroarylalkyl where the heteroaryl moiety contains 1 to 4 heteroatoms selected from N, O, and S as ring-constituting atoms and the alkylene moiety has 1 to 5 carbon atoms; cycloalkylalkyl where the cycloalkyl moiety has 3 to 6 carbon atoms and the alkylene moiety has 1 to 5 carbon atoms; or a C₂₋₆ alkenyl,
X represents O or CH₂,
Y represents C (= O) ; SO₂; C (= O)O ; C (= O)NR¹¹; or an atomic bond, where R¹¹ represents hydrogen; C₁₋₁₀ alkyl; or aralkyl where the aryl moiety has 6 to 10 carbon atoms and the alkylene moiety has 1 to 5 carbon atoms,
provided that the C₁₋₁₀ alkyl as R¹, the alkylene moiety and the cycloalkyl moiety of the cycloalkylalkyl where the cycloalkyl moiety has 3 to 6 carbon atoms and the alkylene moiety has 1 to 5 carbon atoms as R¹; the alkylene moiety of the aralkyl where the aryl moiety has 6 to 10 carbon atoms and the alkylene moiety has 1 to 5 carbon atoms as R¹; and the alkylene moiety of the heteroarylalkyl where the heteroaryl moiety contains 1 to 4 heteroatoms selected from N, O, and S as ring-constituting atoms and the alkylene moiety has 1 to 5 carbon atoms as R¹ may be substituted with at least one substituents selected from 1 to 6 halogens hydroxy; C₁₋₆ alkoxy; C₆₋₁₀ aryloxy; C₁₋₆ alkanoyl; C₁₋₆ alkanoyloxy; carboxyl; alkoxycarbonyl where the alkoxy moiety has 1 to 6 carbon atoms; carbamoyl; alkylcarbamoyl where the alkyl moiety has 1 to 6 carbon atoms; dialkylcarbamoyl where the alkyl moiety has 1 to 6 carbon atoms; alkylsulfonyl where the alkyl moiety has 1 to 6 carbon atoms; aminosulfonyl; alkylsulfinyl where the alkyl moiety has 1 to 6 carbon atoms; alkylthio where the alkyl moiety has 1 to 6 carbon atoms, C₁₋₆ alkoxy substituted with 1 to 6 halogens; and arylcarbonyl where the aryl moiety has 6 to 10 carbon atoms,
the C₆₋₁₀ aryl as R¹; the aryl moiety of the aralkyl where the aryl moiety has 6 to 10 carbon atoms and the alkylene moiety has 1 to 5 carbon atoms as R¹; the aryl moiety of the C₆₋₁₀ aryloxy as R³, R⁴, and R⁵; the aryl moiety of the C₆₋₁₀ arylamino as R³, R⁴, and R⁵; the C₆₋₁₀ aryl as R⁹ and R¹⁰; the heteroaryl containing 1 to 4 heteroatoms selected from N, O, and S as ring-constituting atoms as R⁹ and R¹⁰; the aryl moiety of the aralkyl where the aryl moiety has 6 to 10 carbon atoms and the alkylene moiety has 1 to 5 carbon atoms as R⁹ and R¹⁰; and the heteroaryl moiety of the heteroarylalkyl where the heteroaryl moiety contains 1 to 4 heteroatoms selected from N, O, and S as ring-constituting atoms and the alkylene moiety has 1 to 5 carbon atoms as R⁹ and R¹⁰ may be substituted with at least one substituent selected from
C₁₋₆ alkyl; C₁₋₆ alkoxy, C₁₋₆ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where the alkyl moiety has 1 to 6 carbon atoms; halogen; nitro; cyano; C₁₋₆ alkyl substituted with 1 to 3 halogens; C₁₋₆ alkoxy substituted with 1 to 3 halogens; phenyl; heteroaryl containing 1 to 4 heteroatoms selected from N, O, and S as ring-constituting atoms; phenoxy; phenylalkyl where the alkyl has 1 to 3 carbon atoms; and methylenedioxy,
the heterocyclic ring as R² may have, besides the oxo group, the substituents that the C₆₋₁₀ aryl as R1 mentioned above may have,
when R¹ is C₁₋₁₀ alkyl, it may be substituted with NR11R12, where R¹¹ and R¹², which are the same or different, represent hydrogen; C₁₋₁₀ alkyl; or aralkyl where the aryl moiety has 6 to 10 carbon atoms, and the alkylene moiety has 1 to 5 carbon atoms; or R¹¹, R¹², the nitrogen atom to which R¹¹ and R¹² bind, and optionally, 1 or 2 heteroatoms may combine together to form a 5- to 7-membered ring, and
the alkylene moiety of the aralkyl where the aryl moiety has 6 to 10 carbon atoms, and the alkylene moiety has 1 to 5 carbon atoms as R¹ may be substituted with at least one substituent selected from phenyl, and C₁₋₆ alkyl substituted with 1 to 3 halogens),
a tautomer of the compound, a stereoisomer of the compound, a pharmaceutically acceptable salt thereof, or a solvate thereof.

2. The compound according to claim 1, wherein R¹ represents hydrogen; C₁₋₁₀ alkyl; cycloalkylalkyl where the cycloalkyl moiety has 3 to 6 carbon atoms and the alkylene moiety has 1 to 5 carbon atoms; or aralkyl where the aryl moiety has 6 to 10 carbon atoms and the alkylene moiety has 1 to 5 carbon atoms, a tautomer of the compound, a stereoisomer of the compound, a pharmaceutically acceptable salt thereof, or a solvate thereof.

3. The compound according to claim 1, wherein R¹ represents a cycloalkylalkyl where the cycloalkyl moiety has 3 to 6 carbon atoms and the alkylene moiety has 1 to 5 carbon atoms, a tautomer of the compound, a stereoisomer of the compound, a pharmaceutically acceptable salt thereof, or a solvate thereof.

4. The compound according to claim 1, wherein R¹ represents C₂₋₆ alkyl substituted with hydroxy; C₁₋₆ alkyl substituted with 1 to 6 halogens; or C₂₋₆ alkyl substituted with a C₁₋₆ alkoxy, a tautomer of the compound, a stereoisomer of the compound, a pharmaceutically acceptable salt thereof, or a solvate thereof.

5. The compound according to claim 1, wherein R¹ represents allyl, fluoropropyl, 2-(pyridyl-3-yl) ethyl, 2-(methylsulfonyl) ethyl, or 2-(aminosulfonyl) ethyl, a tautomer of the compound, a stereoisomer of the compound, a pharmaceutically acceptable salt thereof, or a solvate thereof.

6. The compound according to any one of claims 1 to 5, wherein R² represents a 5- to 7-membered saturated heterocycle containing one or two heteroatoms which may be the same or different and are selected from N and O, and three or more carbon atoms as ring-constituting atoms, a tautomer of the compound, a stereoisomer of the compound, a pharmaceutically acceptable salt thereof, or a solvate thereof.

7. The compound according to any one of claims 1 to 5, wherein R² is represented by the following general formula (II), 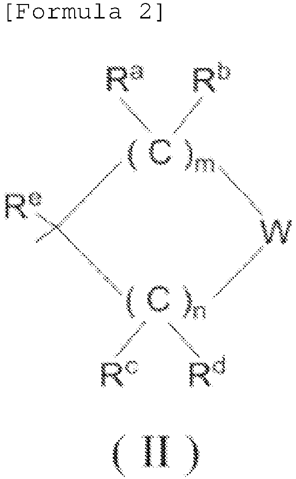
(wherein R^{a} to R^{e}, which are the same or different, represents hydrogen; hydroxy; halogen, C₁₋₆ alkyl, C₁₋₆ alkoxy, C₁₋₆ alkyl substituted with 1 to 3 halogen s, or C₁₋₆ alkoxy substituted with 1 to 3 halogens, or R^{a} and R^{b} or R^{c} and R^{d} combine together to represent an oxo group, wherein
each of C (R^{a}) (R^{b}) s and each of C(R^{c}) (R^{d}) s may be the same or different, respectively, when there are a plurality of C (R^{a}) (R^{b})s and a plurality of C(R^{c}) (R^{d})s,
W represents O or NR^{f}, wherein R^{f} represents hydrogen; C₁₋₆ alkyl; C₁₋₆ alkyl substituted with 1 to 3 halogens; or alkoxycarbonyl where the alkoxy moiety has 1 to 6 carbon atoms, and
m and n each represent 0 or an integer of 1 to 5, and m + n represents 3 to 5),
a tautomer of the compound, a stereoisomer of the compound, a pharmaceutically acceptable salt thereof, or a solvate thereof.

8. The compound according to any one of claims 1 to 5, wherein R² represents pyrrolidinyl, piperidinyl, or tetrahydrofuranyl which may be substituted with 1 to 6 hydroxys which may be the same or different; halogen; C₁₋₆ alkyl; C₁₋₆ alkoxy; C₁₋₆ alkyl substituted with 1 to 3 halogens; C₁₋₆ alkoxy substituted with 1 to 3 halogens; or 1 or 2 oxo groups, a tautomer of the compound, a stereoisomer of the compound, a pharmaceutically acceptable salt thereof, or a solvate thereof.

9. The compound according to any one of claims 1 to 8, wherein X represents CH₂, a tautomer of the compound, a stereoisomer of the compound, a pharmaceutically acceptable salt thereof, or a solvate thereof.

10. The compound according to any one of claims 1 to 9, wherein Y represents C = O, a tautomer of the compound, a stereoisomer of the compound, a pharmaceutically acceptable salt thereof, or a solvate thereof.

11. The compound according to any one of claims 1 to 10, wherein one of R³ and R⁴ is hydroxy and the other is hydrogen, a tautomer of the compound, a stereoisomer of the compound, a pharmaceutically acceptable salt thereof, or a solvate thereof.

12. The compound according to any one of claims 1 to 10, wherein R³ represents halogen; cyano; carbamoyl; C₁₋₆ alkoxy, C₁₋₆ alkanoyloxy; amino; or acylamino where the acyl moiety has 2 to 6 carbon atoms, R⁴ represents hydrogen or hydroxy, and R⁵ represents hydrogen, a tautomer of the compound, a stereoisomer of the compound, a pharmaceutically acceptable salt thereof, or a solvate thereof.

13. The compound according to any one of claims 1 to 10, wherein R³ represents hydroxy; carbamoyl; or a C₁₋₆ alkanoyloxy, R⁴ represents hydrogen, and R⁵ represents hydrogen, a tautomer of the compound, a stereoisomer of the compound, a pharmaceutically acceptable salt thereof, or a solvate thereof.

14. The compound according to any one of claims 1 to 10, wherein R³ represents hydroxy, R⁴ represents hydrogen, and R⁵ represents hydrogen, a tautomer of the compound, a stereoisomer of the compound, a pharmaceutically acceptable salt thereof, or a solvate thereof.

15. The compound according to any one of claims 1 to 10, wherein R³, R⁴, and R⁵ each represent hydrogen, a tautomer of the compound, a stereoisomer of the compound, a pharmaceutically acceptable salt thereof, or a solvate thereof.

16. The compound according to any one of claims 1 to 15, wherein R^{6a}, R^{6b}, R⁷, R⁸, R⁹, and R¹⁰ each represent hydrogen, a tautomer of the compound, a stereoisomer of the compound, a pharmaceutically acceptable salt thereof, or a solvate thereof.

17. A compound selected from (1S,3aR,5aS,6R,11bR,11cS)-3-(L-prolyl)-14-(cyclopropylmethyl)-10-hydroxy-2,3,3a,4,5,6,7,11c-octahydro-1H-6,11b-(epiminoethano)-1,5a-methanonaphtho[1,2-e]indole,
(1S,3aR,5aS,6R,11bR,11cS)-3-(D-prolyl)-14-(cyclopropylmethyl)-10-hydroxy-2,3,3a,4,5,6,7,11c-octahydro-1H-6,11b-(epiminoethano)-1,5a-methanonaphtho[1,2-e]indole,
((1S,3aR,5aS,6R,11bR,11cS)-14-(cyclopropylmethyl)-10-hydroxy-1,2,3a,4,5,6,7,11c-octahydro-3H-6,11b-(epiominoethano)-1,5a-methanonaphtho[1,2-e]indol-3-yl) ((S)-piperidin-2-yl) methanone,
((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((R)-piperidin-2-yl) methanone,
((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((R)-piperidin-3-yl) methanone,
((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) ((S)-piperidin-3-yl) methanone,
((1S,3aR,5aS,6R,11bR,11cS)-14-(cyclopropylmethyl)-10-hydroxy-1,2,3a,4,5,6,7,11c-octahydro-3H-6,11b-(epiminoethano)-1,5a-methanonaphtho[1,2-e]indol-3-yl) (piperidin-4-yl) methanone,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl) piperidine-2,6-dione,
(1S,3aR,5aS,6R,11bR,11cS)-14-(cyclopropylmethyl)-10-hydroxy-3-(methyl-D-prolyl)-2,3,3a,4,5,6,7,11c-octahydro-1H-6,11b-(epiminoethano)-1,5a-methanonaphtho[1,2-e]indol, (R)-5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl) pyrrolidin-2-one,
(R)-5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyrrolidin-2-one,
((1S,3aR,5aS,6R,11bR,11cS)-14-(cyclopropylmethyl)-10-hydroxy-1,2,3a,4,5,6,7,11c-octahydro-3H-6,11b-(epiinoetano)-1,5a-methanonaphtho[1,2-e]indol-3-yl) ((R)-tetrahydrofuran-2-yl) methanone, and
(R)-5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl) dihydrofuran-2(3H)-one,
a tautomer of the compound, a stereoisomer of the compound, a pharmaceutically acceptable salt thereof, or a solvate thereof.

18. A medicament consisting of the compound according to any one of claims 1 to 17, a tautomer of the compound, a stereoisomer of the compound, a pharmaceutically acceptable salt thereof, or a solvate thereof.

19. A pharmaceutical composition comprising the compound according to any one of claims 1 to 17, a tautomer of the compound, a stereoisomer of the compound, a pharmaceutically acceptable salt thereof, or a solvate thereof as an active ingredient.

20. An analgesic comprising the compound according to any one of claims 1 to 17, a tautomer of the compound, a stereoisomer of the compound, a pharmaceutically acceptable salt thereof, or a solvate thereof as an active ingredient.

21. An antidepressant comprising the compound according to any one of claims 1 to 17, a tautomer of the compound, a stereoisomer of the compound, a pharmaceutically acceptable salt thereof, or a solvate thereof as an active ingredient.

22. An anxiolytic comprising the compound according to any one of claims 1 to 17, a tautomer of the compound, a stereoisomer of the compound, a pharmaceutically acceptable salt thereof, or a solvate thereof as an active ingredient.

23. A method for ameliorating, preventing, or treating pain, the method comprising administering an effective amount of the compound according to any one of claims 1 to 17, a tautomer of the compound, a stereoisomer of the compound, a pharmaceutically acceptable salt thereof, or a solvate thereof.

24. A method for ameliorating, preventing, or treating depression, the method comprising administering an effective amount of the compound according to any one of claims 1 to 17, a tautomer of the compound, a stereoisomer of the compound, a pharmaceutically acceptable salt thereof, or a solvate thereof.

25. A method for ameliorating, preventing, or treating anxiety, the method comprising administering an effective amount of the compound according to any one of claims 1 to 17, a tautomer of the compound, a stereoisomer of the compound, a pharmaceutically acceptable salt thereof, or a solvate thereof.

26. Use of the compound according to any one of claims 1 to 17, a tautomer of the compound, a stereoisomer of the compound, a pharmaceutically acceptable salt thereof, or a solvate thereof for ameliorating or treating pain, depression, or anxiety.

27. A method for ameliorating, preventing, or treating pain, depression, or anxiety in a human, the method comprising a step of administering an effective amount of the compound according to any one of claims 1 to 17, a tautomer of the compound, a stereoisomer of the compound, a pharmaceutically acceptable salt thereof, or a solvate thereof to a human.

28. An agent for treating Parkinson's disease, the agent comprising the compound according to any one of claims 1 to 17, a tautomer of the compound, a stereoisomer of the compound, a pharmaceutically acceptable salt thereof, or a solvate as an active ingredient.

29. A method for ameliorating, preventing, or treating Parkinson's disease, the method comprising administering an effective amount of the compound according to any one of claims 1 to 17, a tautomer of the compound, a stereoisomer of the compound, a pharmaceutically acceptable salt thereof, or a solvate thereof.

30. Use of the compound according to any one of claims 1 to 17, a tautomer of the compound, a stereoisomer of the compound, a pharmaceutically acceptable salt thereof, or a solvate thereof for ameliorating or treating Parkinson's disease.

31. A method for ameliorating, preventing, or treating Parkinson's disease in a human, the method comprising a step of administering an effective amount of the compound according to any one of claims 1 to 17, a tautomer of the compound, a stereoisomer of the compound, a pharmaceutically acceptable salt thereof, or a solvate thereof to a human.

32. An agent for treating pollakiuria or urinary incontinence, the agent comprising the compound according to any one of claims 1 to 17, a tautomer of the compound, a stereoisomer of the compound, a pharmaceutically acceptable salt thereof, or a solvate thereof as an active ingredient.

33. A method for ameliorating, preventing, or treating pollakiuria or urinary incontinence, the method comprising administering an effective amount of the compound according to any one of claims 1 to 17, a tautomer of the compound, a stereoisomer of the compound, a pharmaceutically acceptable salt thereof, or a solvate thereof.

34. Use of the compound according to any one of claims 1 to 17, a tautomer of the compound, a stereoisomer of the compound, a pharmaceutically acceptable salt thereof, or a solvate thereof for ameliorating or treating pollakiuria or urinary incontinence.

35. A method for ameliorating, preventing, or treating pollakiuria or urinary incontinence in a human, the method comprising a step of administering an effective amount of the compound according to any one of claims 1 to 17, a tautomer of the compound, a stereoisomer of the compound, a pharmaceutically acceptable salt thereof, or a solvate thereof to a human.

36. An agent for treating glaucoma, the agent comprising the compound according to any one of claims 1 to 17, a tautomer of the compound, a stereoisomer of the compound, a pharmaceutically acceptable salt thereof, or a solvate thereof as an active ingredient.

37. A method for ameliorating, preventing, or treating glaucoma, the method comprising administering an effective amount of the compound according to any one of claims 1 to 17, a tautomer of the compound, a stereoisomer of the compound, a pharmaceutically acceptable salt thereof, or a solvate thereof.

38. Use of the compound according to any one of claims 1 to 17, a tautomer of the compound, a stereoisomer of the compound, a pharmaceutically acceptable salt thereof, or a solvate thereof for ameliorating or treating glaucoma.

39. A method for ameliorating, preventing, or treating glaucoma in a human, the method comprising a step of administering an effective amount of the compound according to any one of claims 1 to 17, a tautomer of the compound, a stereoisomer of the compound, a pharmaceutically acceptable salt thereof, or a solvate thereof to a human.
